# EUROPÄISCHE PATENTSCHRIFT

(11) **EP 1 861 352 B1**
(45) Veröffentlichungstag und Bekanntmachung des Hinweises auf die Patenterteilung: **09.12.2009**
(21) Anmeldenummer: 06723479.9
(22) Anmeldetag: 16.03.2006
(51) Int. Cl.: C07C 59/64, C07C 51/36, C07C 51/41, C07C 67/343, C07C 69/618, C07C 17/16, C07C 19/01

(54) **VERFAHREN ZUR HERSTELLUNG VON OPTISCH AKTIVEN 3-PHENYLPROPIONSÄUREDERIVATEN UND FOLGEPRODUKTE DAVON**
METHOD FOR PRODUCING OPTICALLY ACTIVE 3-PHENYLPROPIONIC ACID DERIVATIVES AND FOLLOW-ON PRODUCTS OF THE LATTER
PROCEDE POUR PRODUIRE DES DERIVES D'ACIDE PROPIONIQUE 3-PHENYLE OPTIQUEMENT ACTIFS ET PRODUITS REACTIONNELS EN DECOULANT

(30) Priorität: 17.03.2005 DE 102005012408; 23.06.2005 DE 102005029228; 05.01.2006 DE 102006000839
(43) Veröffentlichungstag der Anmeldung: 05.12.2007
(73) Patentinhaber: BASF SE, 67056 Ludwigshafen (DE)
(72) Erfinder: HETTCHE, Frank, 69469 Weinheim (DE); VÖLKERT, Martin, 67063 Ludwigshafen (DE); JÄKEL, Christoph, 67117 Limburgerhof (DE); BEY, Olivier, 67150 Niederkirchen (DE)
(86) Internationale Anmeldenummer: PCT/EP2006/002435
(87) Internationale Veröffentlichungsnummer: WO 2006/097314

(56) Entgegenhaltungen:
- WO-A-02/02500
- ISSAKU YAMADA ET AL: "ASYMMETRIC HYDROGENATION OF ACRYLIC ACID DERIVATIVES BY NOVEL CHIRAL RHODIUM-PHOSPHINEDIAMINE COMPLEX CATYLSTS BY SELECTIVE LIGATION BETWEEN TWO AMINO UNITS OF THE LIGAND AND ELECTROSTATIC INTERACTION" JOURNAL OF THE CHEMICAL SOCIETY, PERKIN TRANSACTIONS 1, CHEMICAL SOCIETY. LETCHWORTH, GB, 1990, Seiten 1869-1873, XP002183790 ISSN: 0300-922X

## Beschreibung

Die vorliegende Erfindung betrifft ein Verfahren zur Herstellung von optisch aktiven 3-Phenylpropionsäurederivaten, daraus erhältliche optisch aktive 1-Chlor-3-phenylpropanderivate und dabei erhältliche optisch aktive Zwischenprodukte.

Die asymmetrische Synthese, d. h. Reaktionen, bei denen aus einer prochiralen eine chirale Gruppierung erzeugt wird, so dass die stereoisomeren Produkte (Enantiomere oder Diastereomere) in ungleichen Mengen entstehen, hat vor allem im Bereich der pharmazeutischen Industrie immense Bedeutung gewonnen, da häufig nur ein bestimmtes optisch aktives Isomer therapeutisch aktiv ist. Das gilt auch für die folgende als Synthon A bezeichnete Verbindung 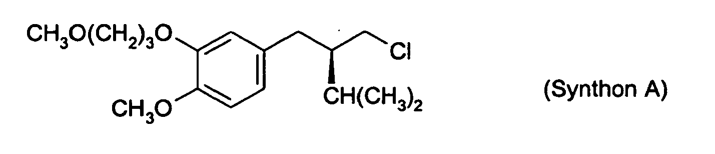 die ein wichtiges Zwischenprodukt bei der Herstellung des Renin-Inhibitors Aliskiren (SPP100) darstellt. Aliskiren ist ein hochwirksamer und selektiver Renin-Inhibitor und als solcher ein wichtiger potentieller Pharmawirkstoff zur Behandlung von Bluthochdruck und verwandten cardiovasculären Erkrankungen (J. M. Wood et al., Biochemical and Biophysical Research Communications 308 (2003) 698 - 705). Es besteht somit ein großer Bedarf an effektiven Synthesewegen zu Systemen vom Typ des Synthon A bzw. seiner optischen Antipoden.

In J. Chem. Soc., Perkin Trans. 1, 1997, S. 1869-1873. wird die asymmetrische Hydrierung von Arylsäurederivaten in Gegenwart von Rhodium-Phosphindiamin-Komplexen als Katalysatoren beschrieben.

In der WO 02/02500 sowie Adv. Synth. Catal. 2003, 345, 160 - 164 ist die Synthese von (R)-2-Alkyl-3-phenylpropionsäuren als Zwischenprodukte bei der Synthon A-Herstellung durch asymmetrische Hydrierung der entsprechenden trans-Acrylsäuren nach folgendem Schema beschrieben

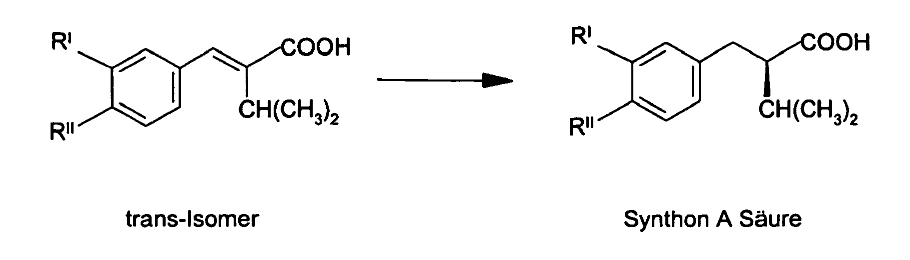

Nachteilig an diesem Verfahren ist die aufwendige Herstellung des trans-Isomers durch wiederholte Extraktion und Kristallisation. Des Weiteren ermöglicht der zur enantioselektiven Hydrierung eingesetzte Katalysator auf Basis eines Phosphinliganden mit Phenylferrocenyl-Rückgrat nur ein niedriges Substrat/Katalysator-Verhältnis (s/c = 5700) bei lediglich 95 % ee, so dass entsprechend hohe Katalysatormengen eingesetzt werden müssen, wodurch das Verfahren wirtschaftlich benachteiligt wird.

Der vorliegenden Erfindung liegt daher die Aufgabe zu Grunde, ein neues Verfahren zur Herstellung von optisch aktiven 3-Phenylpropionsäurederivaten und deren Folgeprodukten, insbesondere von Synthon A, bereitzustellen, welches eine effiziente und kostengünstige technische Synthese erlaubt. Dabei soll es insbesondere möglich sein, ein cis/trans-Isomerengemisch von 3-Phenylacrylsäurederivaten als Zwischenprodukte einzusetzen. Des Weiteren soll bei möglichst hohen Substrat/Katalysator-Verhältnissen, d. h. niedrigen Katalysatormengen (s/c ≥ 10000/1) eine hohe optische Ausbeute (≥ 98 % ee) erzielt werden.

Gelöst wird diese Aufgabe durch ein Verfahren zur Herstellung von optisch aktiven Verbindungen der allgemeinen Formel I 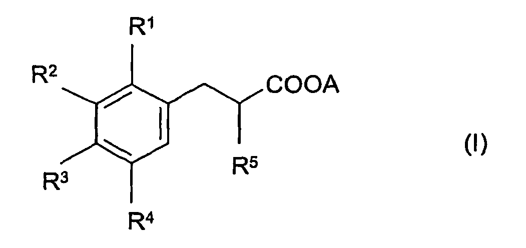 worin
R¹, R², R³ und R⁴ unabhängig voneinander für Wasserstoff, C₁-C₆-Alkyl, Halogen-C₁-C₆-alkyl, Hydroxy-C₁-C₆-alkyl, C₁-C₆-Alkoxy, Hydroxy-C₁-C₆-alkoxy, C₁-C₆-Alkoxy-C₁-C₆-alkyl, Hydroxy-C₁-C₆-alkoxy-C₁-C₆-alkyl, C₁-C₆-Alkoxy-C₁-C₆-alkoxy oder Hydroxy-C₁-C₆-alkoxy-C₁-C₆-alkoxy stehen,
R⁵ für C₁-C₆-Alkyl, C₅-C₈-Cycloalkyl, Phenyl oder Benzyl steht, und
A für Wasserstoff oder ein Kationäquivalent steht, bei dem man
- das cis-Isomer oder ein cis/trans-Isomerengemisch von Verbindungen der allgemeinen Formel II 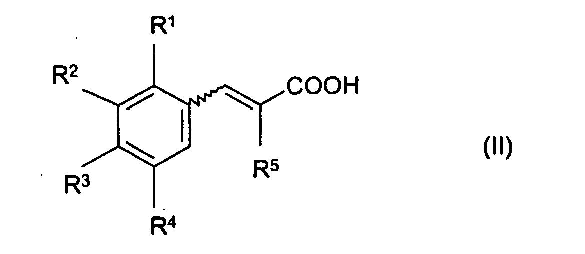
worin R¹ bis R⁵ die zuvor angegebenen Bedeutungen besitzen, einer enantioselektiven Hydrierung in Gegenwart eines chiralen Hydrierungskatalysators, wobei zur Hydrierung ein Übergangsmetallkomplex als Katalysator eingesetzt wird, der als Liganden wenigstens eine Verbindung der Formel 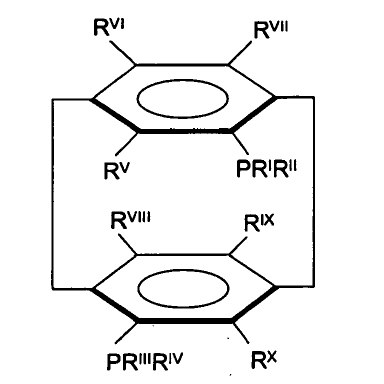 umfasst, worin
R^{I}, R^{II}, R^{III} und R^{IV} unabhängig voneinander für Alkyl, Cycloalkyl, Heterocycloalkyl, Aryl oder Hetaryl stehen, und
R^{V}, R^{VI}, R^{VII}, R^{VIII}, R^{IX} und R^{X} unabhängig voneinander für Wasserstoff, Alkyl, Alkylen-OH, Alkylen-NE¹E², Alkylen-SH, Alkylen-OSiE³E⁴, Cycloalkyl, Heterocycloalkyl, Aryl, Hetaryl, OH, SH, Polyalkylenoxid, Polyalkylenimin, Alkoxy, Halogen, COOH, Carboxylat, SO₃H, Sulfonat, NE¹E², Nitro, Alkoxycarbonyl, Acyl oder Cyano stehen, worin E¹, E², E³ und E⁴ jeweils gleiche oder verschiedene Reste, ausgewählt unter Wasserstoff, Alkyl, Cycloalkyl, Aryl und Alkylaryl bedeuten,
unter Erhalt eines an einem Enantiomeren angereicherten Enantiomerengemischs unterzieht,
- das bei der Hydrierung erhaltene Enantiomerengemisch zur weiteren Enantiomerenanreicherung einer Kristallisation durch Zugabe eines basischen Salzbildners in einem Lösungsmittel unterzieht und den dabei gebildeten, bezüglich eines Stereoisomers angereicherten Feststoff isoliert, und
- gegebenenfalls das isolierte Isomer einer Protonierung oder einem Kationenaustausch unter Erhalt der optisch aktiven Verbindung der Formel I unterzieht.

"Chirale Verbindungen" sind im Rahmen der vorliegenden Erfindung Verbindungen mit wenigstens einem Chiralitätszentrum (d. h. wenigstens einem asymmetrischen Atom, insbesondere wenigstens einem asymmetrischen C-Atom oder P-Atom), mit Chiralitätsachse, Chiralitätsebene oder Schraubenwindung. Der Begriff "chiraler Katalysator" umfasst Katalysatoren, die wenigstens einen chiralen Liganden aufweisen.

"Achirale Verbindungen" sind Verbindungen, die nicht chiral sind.

Unter einer "prochiralen Verbindung" wird eine Verbindung mit wenigstens einem prochiralen Zentrum verstanden. "Asymmetrische Synthese" bezeichnet eine Reaktion, bei der aus einer Verbindung mit wenigstens einem prochiralen Zentrum eine Verbindung mit wenigstens einem Chiralitätszentrum, einer Chiralitätsachse, Chiralitätsebene oder Schraubenwindung erzeugt wird, wobei die stereoisomeren Produkte in ungleichen Mengen entstehen.

"Stereoisomere" sind Verbindungen gleicher Konstitution aber unterschiedlicher Atomanordnung im dreidimensionalen Raum.

"Enantiomere" sind Stereoisomere, die sich zueinander wie Bild zu Spiegelbild verhalten. Der bei einer asymmetrischen Synthese erzielte "Enantiomeren-Überschuss" (enantiomeric excess, ee) ergibt sich dabei nach folgender Formel: ee[%] = (R-S)/(R+S) x 100. R und S sind die Deskriptoren des CIP-Systems für die beiden Enantiomeren und geben die absolute Konfiguration am asymmetrischen Atom wieder. Die enantiomerenreine Verbindung (ee = 100 %) wird auch als "homochirale Verbindung" bezeichnet.

Das erfindungsgemäße Verfahren führt zu Produkten, die bezüglich eines bestimmten Stereoisomers angereichert sind. Der erzielte "Enantiomeren-Überschuss" (ee) beträgt in der Regel wenigstens 98 %.

"Diastereomere" sind Stereoisomere, die nicht enantiomer zueinander sind.

Im Folgenden umfasst der Ausdruck "Alkyl" geradkettige und verzweigte Alkylgruppen. Vorzugsweise handelt es sich dabei um geradkettige oder verzweigte C₁-C₂₀-Alkyl, bevorzugterweise C₁-C₁₂-Alkyl-, besonders bevorzugt C₁-C₈-Alkyl- und ganz besonders bevorzugt C₁-C₆-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten, ausgewählt aus den Gruppen Cycloalkyl, Aryl, Hetaryl, Halogen, NE¹E², NE¹E²E³⁺, COOH, Carboxylat, -SO₃H und Sulfonat, tragen können.

Der Ausdruck "Alkylen" im Sinne der vorliegenden Erfindung steht für geradkettige oder verzweigte Alkandiyl-Gruppen mit vorzugsweise 1 bis 6, insbesondere 1 bis 4 Kohlenstoffatomen. Dazu zählen Methylen (-CH₂-), Ethylen (-CH₂-CH₂-), n-Propylen (-CH₂-CH₂-CH₂-), Isopropylen (-CH₂-CH(CH₃)-) etc.

Der Ausdruck "Cycloalkyl" umfasst im Sinne der vorliegenden Erfindung unsubstituierte als auch substituierte Cycloalkylgruppen, vorzugsweise C₃-C₈-Cycloalkylgruppen, wie Cyclopentyl, Cyclohexyl oder Cycloheptyl, die im Falle einer Substitution, im Allgemeinen 1, 2, 3, 4 oder 5, bevorzugt 1, 2 oder 3 und besonders bevorzugt 1 Substituenten, vorzugsweise ausgewählt aus den für Alkyl genannten Substituenten, tragen können.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, vorzugsweise ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR^{f}, COO⁻M⁺ und NE¹E², bevorzugt Alkyl, tragen können. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl, wobei diese Arylgruppen im Falle einer Substitution im Allgemeinen 1, 2, 3, 4 oder 5, vorzugsweise 1, 2 oder 3 und besonders bevorzugt 1 Substituenten, ausgewählt aus den Gruppen Alkyl, Alkoxy, Carboxyl, Carboxylat, -SO₃H, Sulfonat, NE¹E², Alkylen-NE¹E², Nitro, Cyano oder Halogen, tragen können.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1 ,3,4-Triazolyl und Carbazolyl, wobei diese heterocycloaromatischen Gruppen im Falle einer Substitution im Allgemeinen 1, 2 oder 3 Substituenten, ausgewählt aus den Gruppen Alkyl, Alkoxy, Acyl, Carboxyl, Carboxylat, -SO₃H, Sulfonat, NE¹E², Alkylen-NE¹E² oder Halogen, tragen können.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen NE¹E² stehen vorzugsweise für N,N-Dimethylamino, N-Ethyl-N-methylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenyfamino.

Halogen steht für Fluor, Chlor, Brom und Iod, bevorzugt für Fluor, Chlor und Brom.

Unter einem Kationäquivalent wird ein einwertiges Kation oder der einer positiven Einfachladung entsprechende Anteil eines mehrwertigen Kations verstanden. Vorzugsweise werden Alkalimetall-, insbesondere Na⁺, K⁺-, Li⁺-Ionen oder Onium-Ionen, wie Ammonium-, Mono-, Di-, Tri-, Tetraalkylammonium-, Phosphonium-, Tetraalkylphosphonium- oder Tetraarylphosphonium-Ionen verwendet.

Vorzugsweise stehen die Reste R¹, R², R³ und R⁴ unabhängig voneinander für Wasserstoff, C₁-C₄-Alkyl, wie Methyl, Ethyl, n-Propyl, Isopropyl, n-Butyl und tert.-Butyl, C₁-C₄-Alkoxy, wie Methoxy, Ethoxy, n-Propyloxy oder Isopropyloxy, oder C₁-C₄-Alkoxy-C₁-C₄-alkoxy, wie Methoxyethoxy, Ethoxyethoxy, Methoxy-n-propyloxy, Ethoxy-n-propyloxy.

Bevorzugt stehen R¹ und R⁴ für Wasserstoff und sind R² und R³ unabhängig voneinander ausgewählt unter den zuvor genannten geeigneten und bevorzugten, von Wasserstoff verschiedenen Resten.

Bevorzugt steht R² für Methoxy-n-propyloxy und R³ für Methoxy.

Der Rest R⁵ steht vorzugsweise für C₁-C₆-Alkyl, bevorzugt verzweigtes C₃-C₆-Alkyl und insbesondere für Isopropyl.

Besonders bevorzugt steht A für Wasserstoff oder ein von Ammoniak, primären Aminen, Alkalimetallen und Erdalkalimetallen abgeleitetes Kation. Insbesondere steht A für H⁺, NH₄⁺ oder Li⁺.

Das erfindungsgemäße Verfahren dient in einer speziellen Ausführung zur Herstellung von "Synthon A-Säure" der folgenden Formel 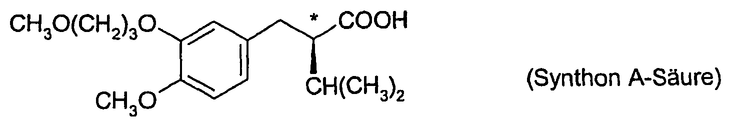 in hoher optischer Reinheit, insbesondere mit einem ee-Wert von wenigstens 98 % (* = Stereozentrum).

Das erfindungsgemäße Verfahren ermöglicht die Herstellung von optisch aktiven Verbindungen der allgemeinen Formel I, wie zuvor beschrieben, ausgehend von dem cis-Isomer oder vorzugsweise einem cis/trans-Isomerengemisch von Verbindungen der allgemeinen Formel II. Vorzugsweise wird ein cis/trans-Isomerengemisch von Verbindungen der allgemeinen Formel II eingesetzt, welches das cis-Isomer in einer Menge von wenigstens 40 %, vorzugsweise im Überschuss enthält. Vorzugsweise enthält das zur Hydrierung eingesetzte Isomerengemisch dann das cis-Isomer in einer Menge von wenigstens 50 Gew.-%, besonders bevorzugt wenigstens 60 Gew.-% und insbesondere wenigstens 70 Gew.-%, bezogen auf das Gesamtgewicht von cis- und trans-Isomer.

Es ist ein charakteristisches Merkmal des erfindungsgemäßen Verfahrens, dass in dem zur enantioselektiven Hydrierung eingesetzten Isomerengemisch von Verbindungen der allgemeinen Formel II auch das trans-Isomer in nicht vernachlässigbaren Mengen enthalten ist. Vorteilhafterweise ermöglicht das Verfahren somit die Herstellung von optisch aktiven Verbindungen der allgemeinen Formel I, ausgehend von cis/trans-Isomerengemischen von Verbindungen der allgemeinen Formel II, wie sie beispielsweise aus Vorläuferverbindungen durch übliche 1,2-Eliminierung, vorzugsweise mit einer gewissen cis-Stereoselektivität, erhältlich sind. Vorzugsweise enthält das zur Hydrierung eingesetzte cis/trans-Isomerengemisch von Verbindungen der allgemeinen Formel II das trans-Isomer in einer Menge von wenigstens 1 Gew.-%, besonders bevorzugt wenigstens 5 Gew.-% und insbesondere wenigstens 10 Gew.-%, bezogen auf das Gesamtgewicht von cis- und trans-Isomer.

Vorteilhafterweise ermöglicht das erfindungsgemäße Verfahren die Herstellung der Verbindungen der Formel I ausgehend von cis/trans-Isomerengemischen in technischen Reinheitsqualitäten. Somit kann auf aufwendige Reinigungsschritte vor der Hydrierung im Allgemeinen verzichtet werden. Vorzugsweise enthalten die eingesetzten cis/trans-Isomerengemisch-Zusammensetzungen wenigstens 80 Gew.-%, besonders bevorzugt wenigstens 85 Gew.-%, an cis- und trans-Isomeren, bezogen auf das Gesamtgewicht der Zusammensetzungen. Weitere enthaltene Komponenten sind z. B. Lösungsmittel sowie Edukte, Zwischenprodukte und Nebenprodukte aus vorhergehenden Reaktionsstufen.

Zur Hydrierung wird vorzugsweise ein chiraler Hydrierungskatalysator eingesetzt, der befähigt ist, das eingesetzte cis/trans-Isomerengemisch unter Bevorzugung des Isomers zu hydrieren, dessen absolute Konfiguration dem (R)-Isomer der Synthon A-Säure entspricht. Ein besonders hoher ee-Wert auf der Stufe der asymmetrischen Hydrierung ist bevorzugt, jedoch nicht allein ausschlaggebend, da nach dem erfindungsgemäßen Verfahren eine weitere Enantiomerenanreicherung im anschließenden Kristallisationsschritt erfolgt. Überraschenderweise wurde jedoch gefunden, dass mit den im Folgenden beschriebenen chiralen Hydrierungskatalysatoren auf Basis planarchiraler Bisphosphane mit Cyclophan-Rückgrat sowohl das cis-Isomer als auch das trans-Isomer in hoher optischer Reinheit zu dem gewünschten Enantiomer, d. h. mit ee-Werten von jeweils mindestens 50 % (z.B. mindestens 70 %), hydriert werden können. Beim Einsatz von cis/trans-Isomerengemischen mit einem cis-Gehalt von wenigstens 70 Gew.-% (bezogen auf das Gesamtgewicht von cis- und trans-Isomer) werden in der Regel ee-Werte von wenigstens 80 % erzielt, bei einem cis-Gehalt von 100 % werden in der Regel ee-Werte von wenigstens 90 % erzielt.

Erfindungsgemäß wird zur Hydrierung ein Übergangsmetallkomplex als Katalysator eingesetzt, der als Liganden wenigstens eine Verbindung der Formel 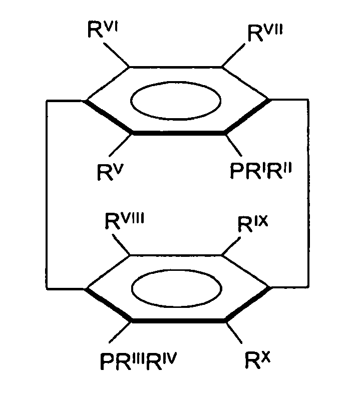 umfasst, worin
R^{I}, R^{II}, R^{III} und R^{IV} unabhängig voneinander für Alkyl, Cycloalkyl, Heterocycloalkyl, Aryl oder Hetaryl stehen, und
R^{V}, R^{VI}, R^{VII}, R^{VIII}, R^{IX} und R^{X} unabhängig voneinander für Wasserstoff, Alkyl, Alkylen-OH, Alkylen-NE¹E², Alkylen-SH, Alkylen-OSiE³E⁴, Cycloalkyl, Heterocycloalkyl, Aryl, Hetaryl, OH, SH, Polyalkylenoxid, Polyalkylenimin, Alkoxy, Halogen, COOH, Carboxylat, SO₃H, Sulfonat, NE¹E², Nitro, Alkoxycarbonyl, Acyl oder Cyano stehen, worin E¹, E², E³ und E⁴ jeweils gleiche oder verschiedene Reste, ausgewählt unter Wasserstoff, Alkyl, Cycloalkyl, Aryl und Alkylaryl bedeuten.

Vorzugsweise sind die an die Phosphoratome gebundenen Reste R^{I}, R^{II}, R^{III} und R^{IV} unabhängig voneinander ausgewählt unter unsubstituierten und substituierten Arylresten. Bevorzugt sind Phenylreste, die 1, 2, 3 oder 4, vorzugsweise 1, 2, oder 3, insbesondere 1 oder 2 Substituenten aufweisen können, die vorzugsweise ausgewählt sind unter Alkyl, Alkoxy, Halogen, SO₃H, Sulfonat, NE¹E², Alkylen-NE¹E², Trifluormethyl, Nitro, Carboxyl, Alkoxycarbonyl, Acyl und Cyano. Bei den Substituenten der Phenyle steht Alkyl vorzugsweise für C₁-C₄-Alkyl und insbesondere für Methyl, Ethyl, Isopropyl und tert.-Butyl, Alkoxy steht vorzugsweise für C₁-C₄-Alkoxy und insbesondere für Methoxy, Alkoxycarbonyl steht vorzugsweise für C₁-C₄-Alkoxycarbonyl. Besonders bevorzugt sind die Reste R^{I}, R^{II}, R^{III} und R^{IV} ausgewählt unter Phenyl, Tolyl, Methoxyphenyl, Methoxyxylyl oder Xylyl, insbesondere unter Phenyl oder Xylyl . Bevorzugt stehen R^{I} bis R^{IV} alle für Phenyl oder alle für Tolyl oder alle für Methoxyphenyl oder alle für Xylyl oder alle für Methoxyxylyl. Die Tolylreste weisen die Methylgruppe vorzugsweise in 4-Position zum Phosphoratom auf. Die Methoxyphenylreste weisen die Methoxygruppe vorzugsweise in 4-Position zum Phosphoratom auf. Die Xylylreste weisen die Methylgruppen vorzugsweise in 3- und 5-Position zum Phosphoratom auf. Die Methoxyxylylreste weisen die Methoxygruppe vorzugsweise in der 4-Position und dabei die Methylgruppen vorzugsweise in 3- und 5-Position zum Phosphoratom auf.

Vorzugsweise steht wenigstens einer der Reste R^{V}, R^{VI} und R^{VII} und/oder einer der Reste R^{VIII}, R^{IX} und R^{X} für einen Wasserstoff verschiedenen Rest und die übrigen Reste für Wasserstoff. Vorzugsweise ist/sind der/die von Wasserstoff verschiedene(n) Rest(e) ausgewählt unter C₁-C₆-Alkyl, C₁-C₄-Alkylen-OH, C₁-C₄-Alkylen-OSi(C₁-C₄-Alkyl)₂, C₁-C₄-Alkoxy, C₁-C₄-Alkylen-OC(Alkyl)₃ und C₁-C₄-Alkylen-OC(Aryl)₃.

In einer bevorzugten Ausführungsform stehen die Reste R^{V} bis R^{X} alle für Wasserstoff. In einer weiteren bevorzugten Ausführungsform ist einer der Reste R^{V}, R^{VI} und R^{VII} und/oder einer der Reste R^{VIII}, R^{IX} und R^{X} ausgewählt unter den Resten der Formeln CH₂OSi(CH(CH₃)₂)₃, CH₂OH, OCH₃, CH₂OC(CH₃)₃ und CH₂OC(C₆H₅)₃, insbesondere unter den Resten der Formeln CH₂OSi(CH(CH₃)₂)₃, CH₂OH, OCH₃ und CH₂OC(C₆H₅)₃.

Besonders bevorzugt als planar-chirale Bisphosphanliganden mit Cyclophan-Rückgrat sind die Liganden der folgenden Formeln 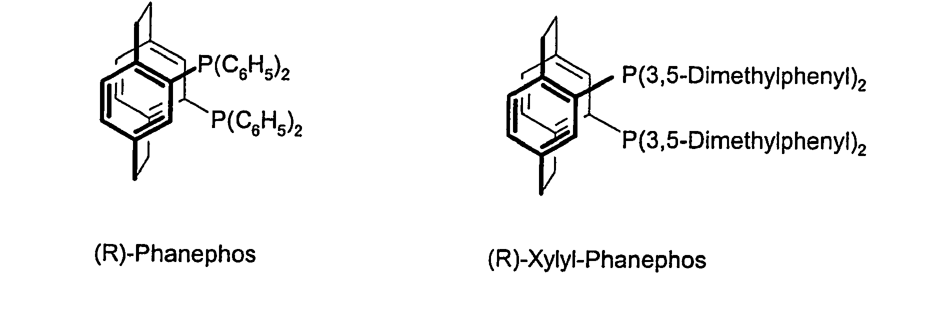 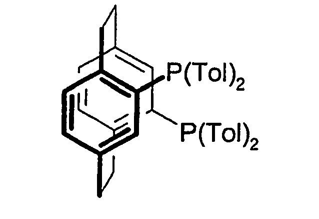 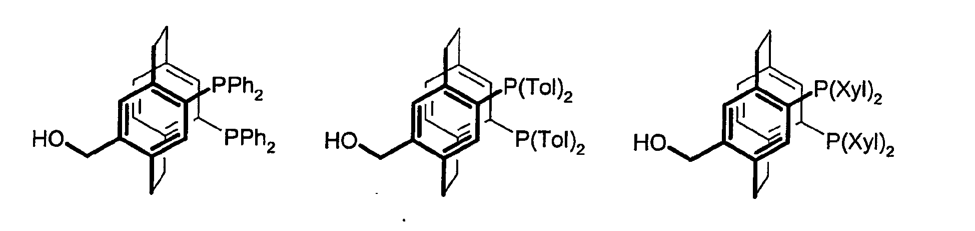 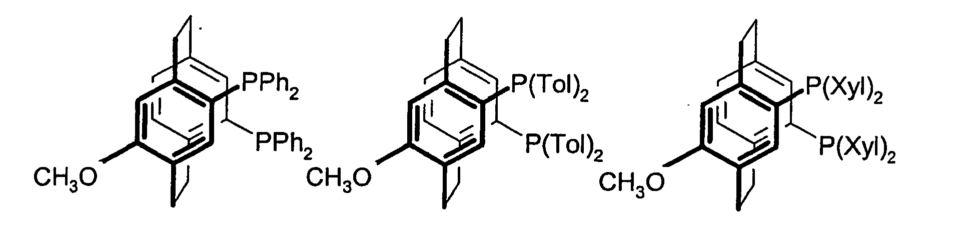 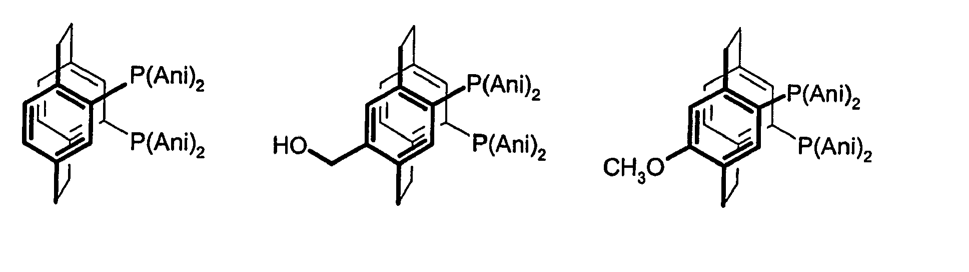 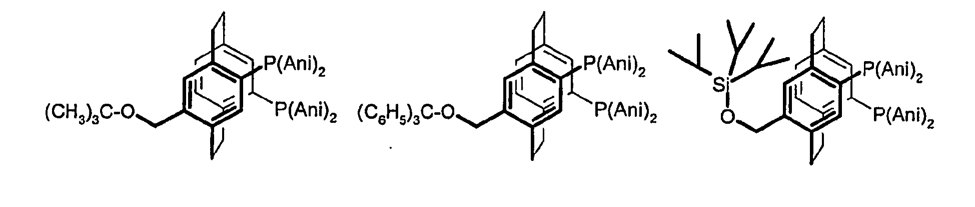 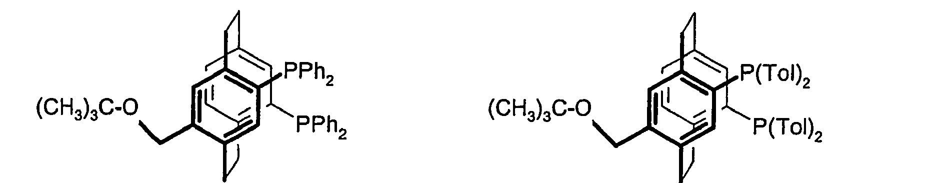 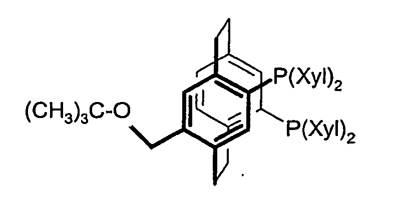 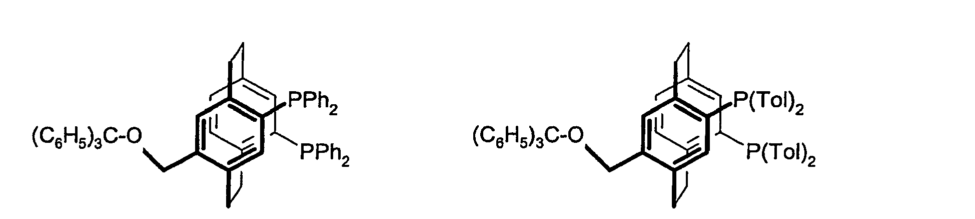 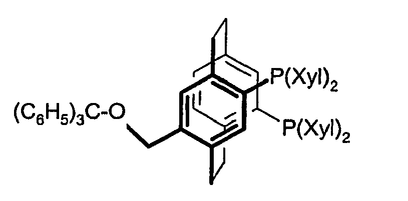 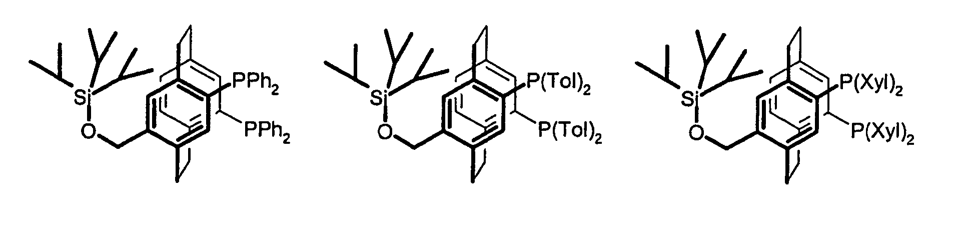 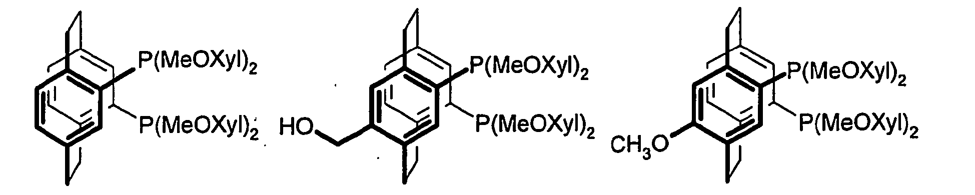 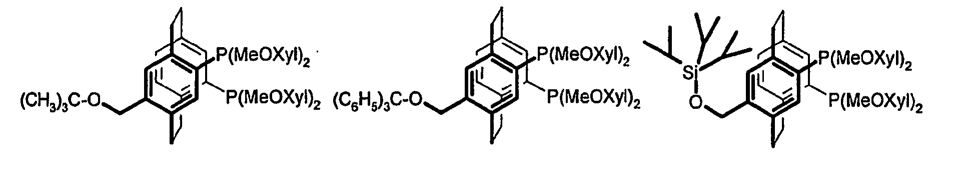 Ph = Phenyl, Tol = 4-Methylphenyl, Xyl = 3,5-Dimethylphenyl, Ani = 4-Methoxyphenyl, MeOXyl = 3,5-Dimethyl-4-methoxyphenyl

Geeignete chirale Paracyclophanphosphine sind dem Fachmann bekannt und beispielsweise von Johnson Matthey Catalysts kommerziell erhältlich.

Die Zuordnung des chiralen Deskriptors "R" zu den abgebildeten Liganden erfolgte übereinstimmend mit P. J. Pye und K. Rossen, Tetrahedron: Asymmetry 9 (1998), S. 539 - 541 und entspricht der kommerziellen Bezeichnung dieser Liganden.

Bevorzugt wird zur enantioselektiven Hydrierung ein Komplex eines Metalls der VIII. Nebengruppe des Periodensystems mit wenigstens einer der zuvor genannten planarchiralen Bisphosphanverbindungen mit Cyclophan-Rückgrat als Liganden eingesetzt. Vorzugsweise ist das Übergangsmetall ausgewählt unter Pd, Pt, Ru, Rh, Ni und Ir. Besonders bevorzugt sind Katalysatoren auf Basis von Rh, Ru und Ir. Insbesondere bevorzugt sind Rh-Katalysatoren.

Phosphin-Metall-Komplexe lassen sich in dem Fachmann bekannter Weise (z. B. Uson, Inorg. Chim. Acta 73, 275 1983, EP-A-0 158 875 , EP-A-437 690) durch Umsetzung der Phosphine mit Komplexen der Metalle, die labile oder hemilabile Liganden enthalten, erhalten. Hierbei können als Metallquellen Komplexe wie etwa Pd₂(dibenzylidenaceton)₃, Pd(Oac)₂, [Rh(COD)Cl]₂, [Rh(COD)₂)]X, Rh(acac)(CO)₂, RuCl₂(COD), Ru(COD)(methallyl)₂, Ru(Ar)Cl₂, Ar = Aryl, sowohl unsubstituiert als auch substituiert, [Ir(COD)Cl]₂, [Ir(COD)₂]X, Ni(allyl)X verwendet werden. Anstatt COD (= 1,5-Cyclooctadien) kann auch NBD (= Norbornadien) verwendet werden. Bevorzugt sind [Rh(COD)Cl]₂, [Rh(COD)₂)]X, Rh(acac)(CO)₂, RuCl₂(COD), Ru(COD)(methallyl)₂, Ru(Ar)Cl₂, Ar = Aryl, sowohl unsubstituiert als auch substituiert, [Ir(COD)Cl]₂ und [Ir(COD)₂]X sowie die entsprechenden Systeme mit NBD als Ersatz von COD. Besonders bevorzugt sind [Rh(COD)₂)]X und [Rh(NBD)₂)]X.

X kann jedes dem Fachmann bekannte generell nutzbare Anion in der asymmetrischen Synthese sein. Beispiele für X sind Halogene wie Cl⁻, Br⁻, I⁻, BF₄⁻, ClO₄⁻, SbF₆⁻, PF₆⁻, CF₃SO₃⁻, BAr₄⁻. Bevorzugt für X sind BF₄⁻, CF₃SO₃⁻, SbF₆⁻, ClO₄⁻, insbesondere BF₄⁻ und CF₃SO₃⁻.

Die Phosphin-Metall-Komplexe können, wie dem Fachmann bekannt ist, entweder vor der eigentlichen Hydrierreaktion im Reaktionsgefäß in situ erzeugt werden oder aber separat erzeugt, isoliert und anschließend eingesetzt werden. Dabei kann es vorkommen, dass sich wenigstens ein Lösungsmittelmolekül an den Phosphin-Metall-Komplex anlagert. Die gängigen Lösungsmittel (z. B. Methanol, Diethylether, Dichlormethan) für die Komplexherstellung sind dem Fachmann bekannt.

Wie dem Fachmann bekannt ist, stellen die Phosphin-Metall- bzw. Phosphin-Metall-LM-Komplexe Präkatalysatoren mit noch mindestens einem labilen oder hemilabilen Liganden dar, aus denen unter den Bedingungen der Hydrierung der eigentliche Katalysator generiert wird.

Als Lösungsmittel für die Hydrierreaktion sind alle dem Fachmann für asymmetrische Hydrierung bekannten Lösungsmittel geeignet. Bevorzugte Lösungsmittel sind niedrige Alkylalkohole wie Methanol, Ethanol, Isopropanol, sowie Toluol, THF, Essigester. Besonders bevorzugt wird in dem erfindungsgemäßen Verfahren Methanol als Lösungsmittel eingesetzt.

Die erfindungsgemäße Hydrierung wird in der Regel bei einer Temperatur von -20 bis 200 °C, bevorzugt bei 0 bis 150 °C und besonders bevorzugt bei 20 bis 120 °C durchgeführt.

Der Wasserstoffdruck kann in einem großen Bereich zwischen 0,1 bar und 325 bar für das erfindungsgemäße Hydrierverfahren variiert werden. Sehr gute Ergebnisse erhält man in einem Druckbereich von 1 bis 300 bar, bevorzugt 5 bis 250 bar.

Vorzugsweise ermöglicht das erfindungsgemäße Verfahren die enantioselektive Hydrierung bei Substrat/Katalysator-Verhältnissen (s/c) von wenigstens 1000 : 1, besonders bevorzugt wenigstens 10000 : 1 und insbesondere wenigstens 30000 : 1. Dabei werden vorteilhafterweise selbst bei Substrat/Katalysator-Verhältnissen von 30000 : 1 noch ee-Werte von wenigstens 80 % erzielt (beim Einsatz eines cis/trans-Isomerengemischs, das wenigstens 70 % cis-Isomer, bezogen auf das Gesamtgewicht von cis-und trans-Isomer, enthält). Dies ist ein entscheidender Vorteil gegenüber den in den bekannten Verfahren eingesetzten Hydrierkatalysatoren.

Die zuvor beschriebenen Hydrierungskatalysatoren (bzw. -präkatalysatoren) können auch in geeigneter Weise, z. B. durch Anbindung über als Ankergruppen geeignete funktionelle Gruppen, Adsorption, Pfropfung, etc. an einen geeigneten Träger, z. B. aus Glas, Kieselgel, Kunstharzen, Polymerträger, etc., immobilisiert werden. Sie eignen sich dann auch für einen Einsatz als Festphasenkatalysatoren. Vorteilhafterweise lässt sich nach diesen Verfahren der Katalysatorverbrauch weiter senken. Die zuvor beschriebenen Katalysatoren eignen sich auch für eine kontinuierliche Reaktionsführung, z. B. nach Immobilisierung, wie zuvor beschrieben, in Form von Festphasenkatalysatoren.

In einer bevorzugten Ausführungsform erfolgt die Hydrierung kontinuierlich. Die kontinuierliche Hydrierung kann in einer oder vorzugsweise in mehreren Reaktionszonen erfolgen. Mehrere Reaktionszonen können von mehreren Reaktoren oder durch räumlich verschiedene Bereiche innerhalb eines Reaktors gebildet werden. Beim Einsatz von mehreren Reaktoren kann es sich jeweils um gleiche oder verschiedene Reaktoren handeln. Diese können jeweils gleiche oder verschiedene Vermischungscharakteristiken aufweisen und/oder durch Einbauten ein- oder mehrfach unterteilt sein. Die Reaktoren können untereinander beliebig verschaltet sein, z. B. parallel oder in Reihe.

Geeignete druckfeste Reaktoren für die Hydrierung sind dem Fachmann bekannt. Dazu zählen die allgemein üblichen Reaktoren für gas-flüssig-Reaktionen, wie z. B. Rohrreaktoren, Rohrbündelreaktoren, Rührkessel, Gasumlaufreaktoren, Blasensäulen, etc., die gegebenenfalls durch Einbauten gefüllt bzw. unterteilt sein können.

Bevorzugt ist ein Verfahren zur kontinuierlichen Hydrierung, bei dem man
i) in eine erste Reaktionszone ein Isomerengemisch von Verbindungen der allgemeinen Formel II und Wasserstoff einspeist und in Gegenwart eines chiralen Hydrierungskatalysators bis zu einem Teilumsatz umsetzt,
ii) aus der ersten Reaktionszone einen Strom entnimmt und in wenigstens einer weiteren Reaktionszone hydriert.

In einer ersten bevorzugten Ausführung wird zur Durchführung des zuvor genannten kaskadierten kontinuierlichen Hydrierverfahrens ein Reaktor eingesetzt, der zwei oder mehr als zwei Reaktionszonen aufweist, die durch Einbauten verwirklicht sind. Bei diesen Einbauten kann es sich beispielsweise um Lochbleche, Füllkörper, Packungen oder Kombinationen davon handeln. In einer zweiten bevorzugten Ausführungsform wird zur Durchführung des zuvor genannten kaskadierten kontinuierlichen Hydrierverfahrens ein Reaktionssystem eingesetzt, das aus zwei in Reihe geschalteten Reaktoren besteht.

Die Temperatur bei der Hydrierung liegt in allen Reaktionszonen im Allgemeinen in einem Bereich von etwa 10 bis 200 °C, bevorzugt 20 bis 150 °C. Gewünschtenfalls kann in der zweiten Reaktionszone eine andere, vorzugsweise eine höhere Temperatur als in der ersten Reaktionszone, bzw. in jeder nachfolgenden Reaktionszone eine höhere Temperatur als in einer vorhergehenden Reaktionszone eingestellt werden, z. B. um einen möglichst vollständigen Umsatz bei der Hydrierung zu erzielen. Die Umsetzung wird in allen Reaktionszonen vorzugsweise bei einem Wasserstoffdruck in einem Bereich von etwa 1 bis 300 bar, bevorzugt 5 bis 250 bar, durchgeführt. Gewünschtenfalls kann in der zweiten bzw. einer folgenden Reaktionszone ein anderer, z. B. ein höherer Wasserstoffdruck als in der ersten bzw. einer vorhergehenden Reaktionszone eingestellt werden.

Das Reaktorvolumen und/oder die Verweilzeit der ersten Reaktionszone werden so gewählt, dass im Allgemeinen mindestens etwa 10 % des eingespeisten Isomerengemischs umgesetzt werden. Bevorzugt beträgt der auf das eingespeiste Isomerengemisch bezogene Umsatz in der ersten Reaktionszone mindestens 80 %.

Zur Abfuhr der bei der exothermen Hydrierung entstehenden Reaktionswärme kann die erste und/oder die folgende(n) Reaktionszone(n) mit einer Kühlvorrichtung versehen sein. Die Abfuhr der Reaktionswärme kann durch Kühlung eines externen Umlaufstroms oder durch interne Kühlung in wenigstens einer der Reaktionszonen erfolgen. Zur internen Kühlung können die dafür üblichen Vorrichtungen, im Allgemeinen Hohlkörpermodule, wie Field-Rohre, Rohrschlangen, Wärmetauscherplatten, etc. eingesetzt werden. Sofern das in der zweiten oder einer nachfolgenden Reaktionszone hydrierte Reaktionsgemisch nur noch so geringe Anteile an hydrierbaren Verbindungen aufweist, dass die bei der Reaktion auftretende Wärmetönung nicht ausreicht, die erwünschte Temperatur in der Reaktionszone zu halten, kann auch eine Erwärmung der zweiten oder einer nachfolgenden Reaktionszone erforderlich sein. Diese kann analog der zuvor beschriebenen Abfuhr der Reaktionswärme durch Erwärmung eines externen Umlaufstroms oder durch interne Erwärmung in der Reaktionszone erfolgen. In einer geeigneten Ausführung kann zur Temperierung der zweiten oder einer nachfolgenden Reaktionszone die Reaktionswärme aus der ersten bzw. einer vorhergehenden Reaktionszone verwendet werden.

Des Weiteren kann zur Erwärmung der Edukte die dem Reaktionsgemisch entzogene Reaktionswärme verwendet werden. In einer speziellen Ausgestaltung des Verfahrens wird eine Reaktorkaskade aus zwei in Reihe geschalteten Reaktoren eingesetzt, wobei die Umsetzung in dem zweiten Reaktor adiabatisch durchgeführt wird. Dieser Begriff wird im Rahmen der vorliegenden Erfindung im technischen und nicht im physikochemischen Sinne verstanden. So erfährt das Reaktionsgemisch beim Strömen durch den zweiten Reaktor auf Grund der exothermen Hydrierungsreaktion eine Temperaturerhöhung. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem zweiten Reaktor abgeführt, abgesehen von einem Restanteil, der durch natürliche Wärmeleitung und Wärmeabstrahlung vom Reaktor an die Umgebung abgegeben wird.

Zur Verringerung des Temperaturgradienten über alle Reaktionszonen kann beim Einsatz einer externen Kühlung der Zuführstrom der Nachreaktoren nach dem externen Wärmetauscher abgezogen werden. Dadurch sinkt die Eintrittstemperatur des Nachreaktors auf die Austrittstemperatur des Wärmetauschers und wird unterhalb der Austrittstemperatur des Hauptreaktors liegen. Somit verringert sich die Austrittstemperatur der nachfolgenden Reaktionszone.

In einer Ausführungsform kann in wenigstens einer der eingesetzten Reaktionszonen oder in dem Reaktorsystem insgesamt eine zusätzliche Durchmischung erfolgen. Eine zusätzliche Durchmischung ist insbesondere vorteilhaft, wenn die Hydrierung bei großen Verweilzeiten des Reaktionsgemischs erfolgt. Zur Durchmischung können z. B. die in die Reaktionszone eingeführten Ströme eingesetzt werden, indem man diese über geeignete Mischvorrichtungen, wie Düsen, in die jeweilige Reaktionszone einführt. Zur Durchmischung können auch in einem externen Kreislauf geführte Ströme aus der jeweiligen Reaktionszone eingesetzt werden. In einer speziellen Ausführung weist das Reaktorsystem einen Gasraum auf, dem ein gasförmiger Strom entnommen und, gegebenenfalls nach Temperieren in einem Wärmetauscher, über eine geeignete Mischvorrichtung, vorzugsweise eine Düse, dem flüssigen Reaktionsgemisch wieder zudosiert wird (Kreisgasverfahren). Die Ansaugung des Kreisgases aus dem Gasraum erfolgt vorzugsweise durch die Mischvorrichtung, die in Form eines Ejektors ausgebildet ist.

Die Einspeisung des für die Hydrierung benötigten Wasserstoffs kann in die erste sowie zusätzlich in die folgende(n) Reaktionszone(n) erfolgen. Vorzugsweise erfolgt die Einspeisung von Wasserstoff nur in die erste Reaktionszone.

Der Austrag aus der Hydrierung kann vor der Enantiomerenanreicherung einer ein- oder mehrstufigen Trennoperation unterzogen werden, wobei zumindest ein die Hauptmenge des Hydrierungsprodukts enthaltender Strom und gegebenenfalls zusätzlich ein den Hydrierungskatalysator enthaltender Strom erhalten werden. Dazu kann der Austrag aus der Hydrierung zunächst einer Entgasung zur Isolierung überschüssigen Wasserstoffs unterzogen werden. Die resultierende Flüssigphase, die das Hydrierungsprodukt, den Katalysator sowie gegebenenfalls eingesetztes Lösungsmittel enthält, kann dann nach üblichen, dem Fachmann bekannten Verfahren weiter aufgetrennt werden. Dazu zählt die thermische Auftrennung durch Destillation oder eine extraktive Auftrennung.

Zur weiteren Aufarbeitung wird das bei der Hydrierung erhaltene Enantiomerengemisch einer Enantiomeren-anreichernden Kristallisation unter Zugabe eines basischen Salzbildners unterzogen. Geeignete basische Salzbildner sind übliche, dem Fachmann bekannte asymmetrische Amine, wie z. B. (R)-Phenethylamin. Beim Einsatz solcher asymmetrischen Amine werden in der Regel ee-Werte von etwa 99,5 % erzielt. Überraschenderweise wurde gefunden, dass zur Enantiomeren-anreichernden Kristallisation auch achirale basische Verbindungen als Salzbildner eingesetzt werden können. Vorzugsweise sind diese ausgewählt unter Ammoniak, primären Aminen, wie Methylamin, Ethylamin, n-Propylamin, Isopropylamin, n-Butylamin, n-Pentylamin, n-Hexylamin, Cyclohexylamin, Alkalihydroxiden, wie KOH, NaOH, LiOH, und Erdalkalihydroxiden, wie Ca(OH)₂ und Mg(OH)₂.

Vorzugsweise erfolgt die Enantiomeren-anreichernde Kristallisation aus einem Lösungsmittel, das ausgewählt ist unter organischen Lösungsmitteln, vorzugsweise wassermischbaren organischen Lösungsmitteln, Lösungsmittelgemischen sowie Gemischen aus wassermischbaren organischen Lösungsmitteln und Wasser. Geeignete organische Lösungsmittel sind einwertige Alkohole, wie Methanol, Ethanol, n-Propanol, Isopropanol, n-Butanol, Cyclohexanol; Polyole, wie Ethylenglycol und Glycerin; Ether und Glycolether, wie Dieethylether, Dibutylether, Anisol, Dioxan, Tetrahydrofuran, Mono-, Di-, Tri- und Polyalkylenglycolether; Ketone, wie Aceton, Butanon, Cyclohexanon; Gemische der zuvor genannten Lösungsmittel sowie Gemische aus einzelnen oder mehreren der zuvor genannten Lösungsmittel mit Wasser. Besonders bevorzugt werden als Lösungsmittel Alkanole und Alkanol-Wasser-Gemische und speziell Isopropanol und Isopropanol-Wasser-Gemische eingesetzt.

In einer geeigneten Vorgehensweise kann das Produkt der Enantiomeren-anreichernden Hydrierung in dem Lösungsmittel gelöst oder suspendiert und anschließend der Salzbildner als Lösung in dem gleichen oder einem verschiedenen Lösungsmittel oder in fester Form zugegeben werden. So ist es beispielsweise möglich, das Produkt der Hydrierung in einer zur vollständigen Lösung ausreichenden Lösungsmittelmenge zu lösen und anschließend eine wässrige Lösung des Salzbildners zuzusetzen. In einer bevorzugten Ausführung wird das Hydrierungsprodukt in Isopropanol gelöst und anschließend mit einer wässrigen Ammoniaklösung versetzt. Geeignet ist beispielsweise eine 20 bis 30%ige wässrige Ammoniaklösung. In einer weiteren bevorzugten Ausführung wird das Hydrierungsprodukt in Isopropanol gelöst und mit festem LiOH versetzt und die resultierende Suspension anschließend gerührt. Eine ausreichende Rührzeit liegt beispielsweise im Bereich von etwa 10 Minuten bis 12 Stunden, vorzugsweise 20 Minuten bis 6 Stunden, insbesondere 30 Minuten bis 3 Stunden.

Die Temperatur bei der Enantiomeren-anreichernden Kristallisation liegt im Allgemeinen im Bereich zwischen Schmelzpunkt und Siedepunkt des eingesetzten Lösungsmittels bzw. Lösungsmittelgemischs. In einer geeigneten Ausführungsform kann die Temperatur im Verlauf der Kristallisation ein- oder mehrfach erhöht und/oder abgesenkt werden, um die Kristallbildung zu initiieren und/oder die Fällung des gewünschten Enantiomers zu vervollständigen.

Vorteilhafterweise weist der nach der Enantiomeren-anreichernden Kristallisation isolierte Feststoff einen ee-Wert von mindestens 98 %, besonders bevorzugt mindestens 99 % und insbesondere größer 99,5 % auf.

Gewünschtenfalls können die bei der Enantiomeren-anreichernden Kristallisation isolierten Verbindungen einer Protonierung oder einem Kationenaustausch unterzogen werden. So ist es beispielsweise möglich, zur Protonierung unter Erhalt einer optisch aktiven Verbindung der Formel I, worin A für Wasserstoff steht, das Produkt der Kristallisation mit einer geeigneten Säure, vorzugsweise einer Mineralsäure, wie HCl, H₂SO₄, H₃PO₄ in Kontakt zu bringen. In einer geeigneten Vorgehensweise wird das Produkt der Kristallisation in Wasser gelöst oder suspendiert und anschließend der pH-Wert durch Säurezugabe auf etwa 0 bis 4, vorzugsweise etwa 1, eingestellt. Zur Isolierung der freien Säure ist es möglich, die angesäuerte Lösung oder Suspension mit einem geeigneten organischen Lösungsmittel, z. B. einem Ether, wie Methylbutylether, einem Kohlenwasserstoff oder Kohlenwasserstoffgemisch, z. B. einem Alkan, wie Pentan, Hexan, Heptan, oder einem Alkangemisch, Ligroin oder Petrolether, oder Aromaten, wie Toluol, zu extrahieren. Ein bevorzugtes Extraktionsmittel ist Toluol. Bei dieser Vorgehensweise kann die Säure nahezu quantitativ erhalten werden, wobei auch der ee-Wert erhalten bleibt.

In einer bevorzugten Ausführung ermöglicht das erfindungsgemäße Verfahren die Herstellung von optisch aktiven Verbindungen der Formel I mit der folgenden absoluten Konfiguration 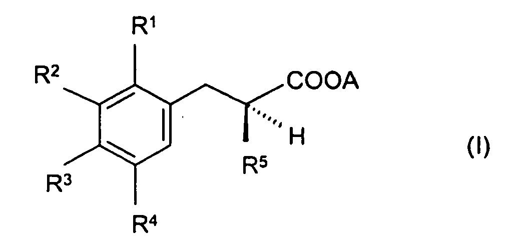 wobei R¹ bis R⁵ und A die zuvor angegebenen Bedeutungen besitzen. Das erfindungsgemäße Verfahren eignet sich somit in besonders vorteilhafter Weise zur Herstellung von Zwischenprodukten, die sich zur Weiterverarbeitung zu Synthon A und Synthon A-Derivaten eignen.

Die Erfindung ermöglicht die Herstellung von optisch aktiven Verbindungen der allgemeinen Formel III 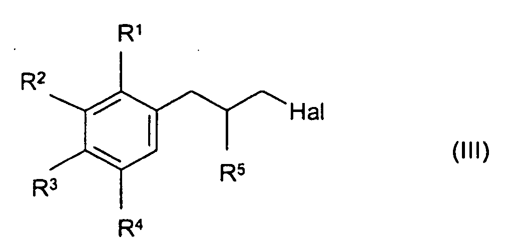 worin R¹ bis R⁵ die zuvor angegebenen Bedeutungen besitzen und Hal für Cl, Br oder I steht, wobei man
- eine Verbindung der allgemeinen Formel I, wie zuvor definiert, für den Fall, dass A nicht für ein Metallkation oder Proton steht, durch Protonierung in die Säure überführt,
- die, gegebenenfalls nach Protonierung erhaltene, Säure oder ein Metallsalz davon einer Reduktion unter Erhalt eines Alkohols der allgemeinen Formel IV 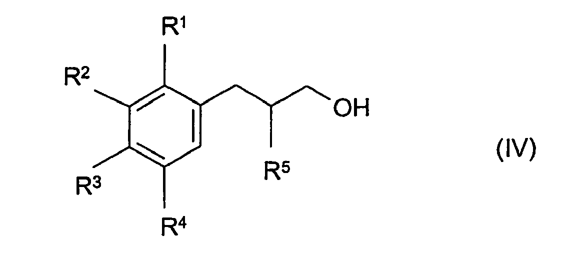 unterzieht, worin R¹ bis R⁵ die zuvor angegebenen Bedeutungen besitzen und
- den Alkohol der Formel IV einer Halodehydroxylierung unter Erhalt der optisch aktiven Verbindung der Formel III unterzieht.

Zur Reduktion wird die Verbindung der Formel I vorzugsweise in Form der freien Säure eingesetzt. Um Verbindungen der Formel I, worin A für ein von Protonen verschiedenes Kationäquivalent steht, in die freie Säure zu überführen, kann wie zuvor beschrieben verfahren werden. Vorzugsweise wird dazu die Verbindung der Formel I mit einer Mineralsäure, wie HCl, H₂SO₄ oder H₃PO₄ in Kontakt gebracht. Vorzugsweise erfolgt die Protonierung der Verbindung der Formel I in einem wässrigen Medium. Die Isolierung der freien Säure erfolgt vorzugsweise mit einem geeigneten organischen Lösungsmittel, vorzugsweise durch Extraktion mit einem nicht wassermischbaren oder nur gering wassermischbaren Lösungsmittel. Geeignete Lösungsmittel sind z. B. Ether, wie Diethylether, Methylbutylether und Methyl-tert.-butylether, die zuvor genannten Kohlenwasserstoffe oder Kohlenwasserstoffgemische, Aromaten, wie Toluol, sowie halogenierte Aromaten, wie Dichlormethan, Chloroform, Tetrachlorkohlenstoff und 1,2-Dichlorethan. Vorzugsweise erfolgt die Isolierung und/oder Reinigung der Säure durch Extraktion einer die Säure enthaltenden organischen Phase mit einer wässrigen Phase. Bei einer solchen Vorgehensweise kann die Säure, wie zuvor beschrieben, nahezu quantitativ erhalten werden, wobei der ee-Wert ebenfalls erhalten bleibt.

Zur Reduktion der Verbindungen der Formel I, worin A für ein Proton oder ein Metallkation steht, eignen sich prinzipiell die zur Reduktion von Carbonsäuren zu Alkoholen üblichen Reagenzien, wie komplexe Hydride, sowie katalytische Hydrierungsverfahren mit molekularem Wasserstoff. Geeignete Verfahren und Reaktionsbedingungen sind in J. March, Advanced Organic Chemistry, 4. Auflage, Verlag John Wiley & Sons (1992), S. 1212 sowie Tabelle 19.5, S. 1208, beschrieben, worauf hier Bezug genommen wird. Bevorzugt werden komplexe Hydride, wie LiAlH₄, AlH₃, LiAlH(OCH₃)₃, LiAlH(O-t.-C₄H₉)₃, (i.-C₄H₉)₂AlH (= DIBALH), NaAl(C₂H₅)₂H₂, NaAl(CH₃OC₂H₄O)₂H₂ (= Vitride), etc., eingesetzt.

Die Umwandlung des bei der Reduktion erhaltenen Alkohols der allgemeinen Formel IV in ein Alkylhalogenid kann nach üblichen, dem Fachmann bekannten Verfahren erfolgen. Geeignete Verfahren sind in J. March, Advanced Organic Chemistry, 4. Auflage, Verlag John Wiley & Sons (1992), S. 431 - 433 beschrieben, worauf hier Bezug genommen wird. Vorzugsweise wird zur Halodehydroxylierung eine Halogenwasserstoffsäure, wie HCl, HBr, HI, oder ein anorganisches Säurehalogenid, wie SOCl₂, PCl₅, PCl₃, POCl_{3,} etc., eingesetzt. Vorzugsweise wird der Alkohol in das entsprechende Alkylchlorid (Hal = Cl) umgesetzt. Dabei handelt es sich in einer besonders bevorzugten Ausführungsform des erfindungsgemäßen Verfahrens um das Synthon A.

Die Verbindung der Formel III kann gewünschtenfalls einer abschließenden Reinigung nach üblichen, dem Fachmann bekannten Verfahren, z. B. durch Umkristallisation aus einem geeigneten Lösungsmittel, unterzogen werden.

Das erfindungsgemäße Verfahren kann vorteilhaft als Teil einer Gesamtsynthese zur Herstellung von Synthon A und Synthon A-Derivaten eingesetzt werden. Gegenstand der Erfindung ist daher auch ein Verfahren, wie zuvor definiert, bei dem man
a) einen aromatischen Aldehyd der allgemeinen Formel V 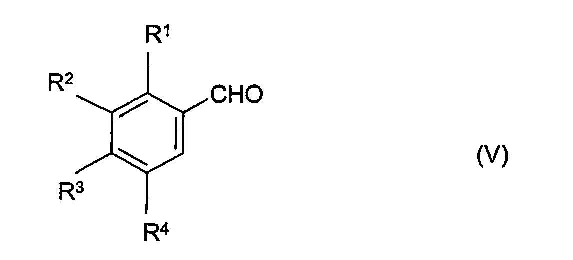 worin R¹ bis R⁴ die zuvor angegebenen Bedeutungen besitzen, mit einem Carbonsäureester der allgemeinen Formel VI

   R⁵-CH₂-COOR⁷ (VI)

   worin R⁵ die in Anspruch 1 angegebenen Bedeutungen besitzt und R⁷ für Alkyl, Cycloalkyl, Aryl oder Alkylaryl steht, unter Erhalt von Verbindungen der allgemeinen Formel VII 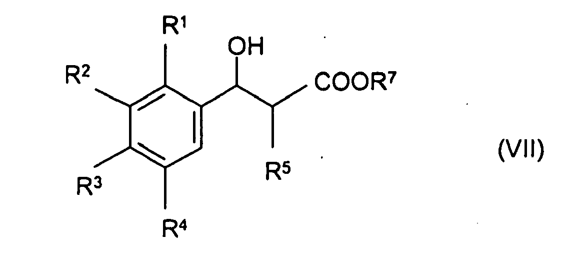 umsetzt,
b) in den Verbindungen der Formel VII die Hydroxylgruppe in eine bessere Abgangsgruppe überführt und einer Eliminierung unter Erhalt von Verbindungen der allgemeinen Formel VIII 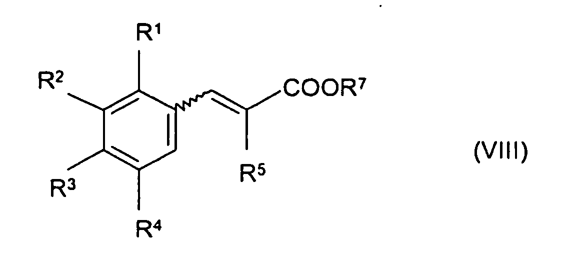 unterzieht,
c) die Verbindungen der Formel VIII einer Esterhydrolyse unter Erhalt von Verbindungen der allgemeinen Formel II 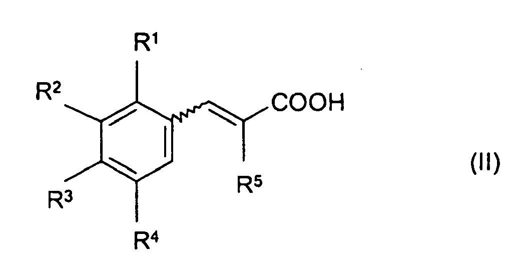 unterzieht,
d) die Verbindungen der Formel II einer enantioselektiven Hydrierung in Gegenwart eines erfindungsgemäß eingesetzten chiralen Hydrierungskatalysators unterzieht, unter Erhalt eines an einem Enantiomeren angereicherten Enantiomerengemischs,
e) das bei der Hydrierung in Schritt d) erhaltene Enantiomerengemisch zur weiteren Enantiomerenanreicherung einer Kristallisation durch Zugabe eines basischen Salzbildners in einem Lösungsmittel unterzieht und den dabei gebildeten, bezüglich eines Stereoisomers angereicherten Feststoff isoliert,
f) gegebenenfalls das in Schritt e) isolierte Isomer einer Protonierung oder einem Kationenaustausch unter Erhalt der optisch aktiven Verbindung der Formel I undterzieht,
g) für den Fall, dass in der Verbindung der Formel I der Rest A für ein von Wasserstoff und Metallkationen verschiedenes Kationäquivalent steht, diesen einer Protonierung unterzieht,
h) die Säure oder das Metallsalz davon einer Reduktion unter Erhalt eines Alkohols der allgemeinen Formel IV 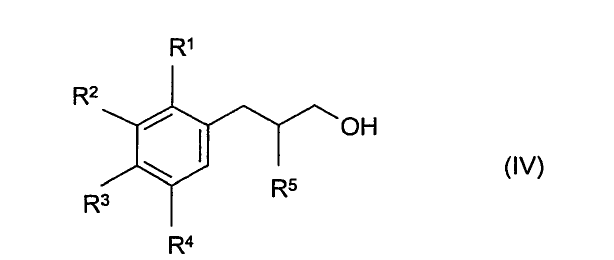 unterzieht, und
i) den Alkohol der Formel IV einer Halodehydroxylierung unter Erhalt der optisch aktiven Verbindung der Formel III 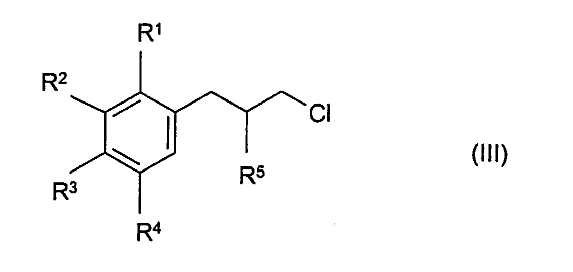 unterzieht.

Die nach dem erfindungsgemäßen Verfahren als Zwischenprodukte erhaltenen optisch aktiven Verbindungen der allgemeinen Formel I 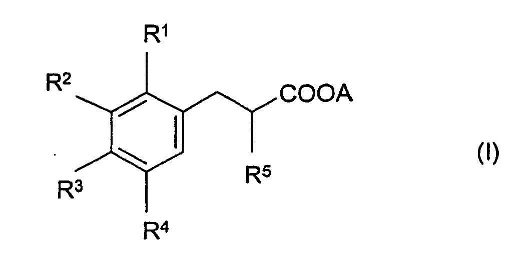 worin R¹ bis R⁵ die zuvor angegebene Bedeutung besitzen und A für ein von Ammoniak, primären Aminen, Alkalimetallen und Erdalkalimetallen abgeleitetes Kation stehen, sind neu und ebenfalls Gegenstand der Erfindung. Bevorzugt steht in den Verbindungen der Formel I der Rest R⁵ für einen verzweigten C₃-C₈-Alkylrest und insbesondere für Isopropyl. Die erfindungsgemäßen Verbindungen weisen vorzugsweise die folgende Formel auf:

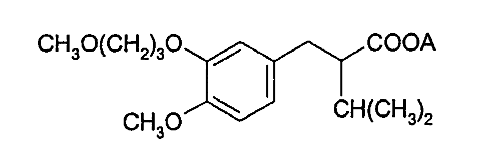

Insbesondere handelt es sich um Verbindungen, wobei A für NH₄⁺ oder Li⁺ steht.

Die als Edukt in Schritt a) eingesetzten aromatischen Aldehyde der Formel V sind kommerziell erhältlich oder können nach üblichen, dem Fachmann bekannten Verfahren hergestellt werden. In einer geeigneten Ausführung zur Herstellung von "Synthon A" kann man beispielsweise ausgehend von 3-Hydroxy-4-methoxybenzaldehyd (Isovanillin) die Hydroxyfunktion einer Veretherung unter Erhalt von 3-(3-Methoxypropoxy)-4-methoxybenzaldehyd als Verbindung der Formel V unterziehen.

Geeignete Verfahrensbedingungen zur Umsetzung aromatischer Aldehyde mit Carbonsäureestern, welche über acide Wasserstoffatome verfügen im Sinne einer Aldolreaktion, sind z. B. in J. March, Advanced Organic Chemistry, 4. Auflage, Verlag John Wiley & Sons (1992), S. 944 - 951 beschrieben, worauf hier Bezug genommen wird. Die Reaktion erfolgt in der Regel in Gegenwart einer starken Base, die vorzugsweise ausgewählt ist unter Alkalimetallalkoholaten, wie Natriummethanolat, Kaliummethanolat, Kalium-tert.-butanolat, Alkalimetallhydriden, wie Natriumhydrid, sekundären Amiden, wie Lithiumamid, Lithiumdiisopropylamid, etc. Die Reaktion erfolgt vorzugsweise bei einer Temperatur im Bereich von -80 bis +30 °C, insbesondere von -60 bis +20 °C. Geeignete Lösungsmittel sind z. B. Ether, wie Diethylether, Tetrahydrofuran und Dioxan, Aromaten, wie Benzol, Toluol und Xylol, etc.

Die Dehydratisierung in Reaktionsschritt b) ist ebenfalls prinzipiell bekannt. Vorzugsweise erfolgt die Überführung der Hydroxylgruppe in eine bessere Abgangsgruppe durch Umsetzung mit einer Sulfonsäure oder einem Derivat davon, wie Benzolsulfonsäure, Toluolsulfonsäure, Methylsulfonsäure, Trifluormethylsulfonsäure oder einem Derivat, z. B. einem Halogenid, davon. In einer bevorzugten Ausführung erfolgt die Dehydratisierung in einem zur Bildung niedrig siedender Azeotrope mit Wasser befähigtem Lösungsmittel, wie Benzol oder vorzugsweise Toluol. Die Entfernung des bei der Reaktion gebildeten Wassers kann dann durch azeotrope Destillation (Auskreisen) nach üblichen, dem Fachmann bekannten Verfahren erfolgen. Bei dieser Vorgehensweise ist es möglich, die zur Bildung der Abgangsgruppe befähigte Säure lediglich in katalytischen Mengen einzusetzen. Es wurde gefunden, dass bei dieser Vorgehensweise vorteilhafterweise cis/trans-Isomerengemische von Verbindungen der Formel VIII erhalten werden, welche das cis-Isomer im Überschuss enthalten.

Verfahren zur Hydrolyse von Carbonsäureestem (Schritt c)) zu den entsprechenden Carbonsäuren oder zu Salzen davon sind ebenfalls prinzipiell bekannt und z. B. in J. March, Advanced Organic Chemistry, 4. Auflage, Verlag John Wiley & Sons (1992), S. 378 - 383 beschrieben, worauf hier Bezug genommen wird. Die Esterhydrolyse kann prinzipiell sauer oder basisch erfolgen.

Bezüglich der Verfahrensschritte d) bis i) wird auf die vorherigen Ausführungen zu geeigneten und bevorzugten Verfahrensbedingungen in vollem Umfang Bezug genommen.

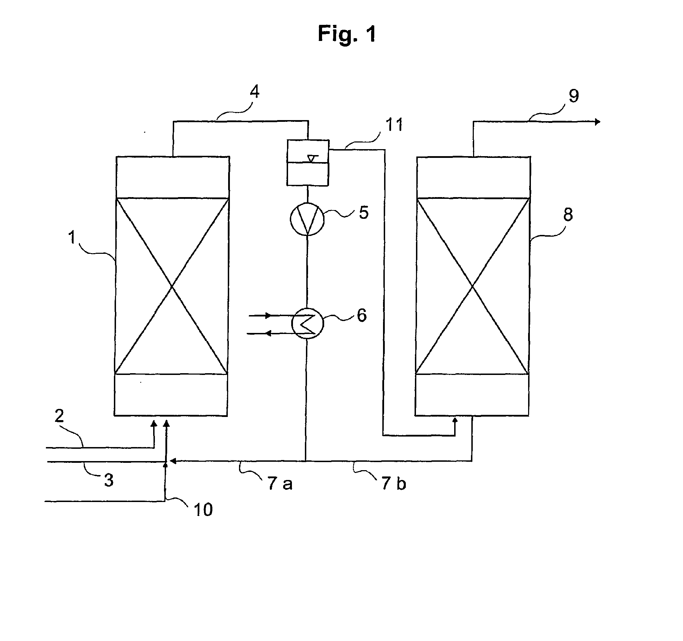

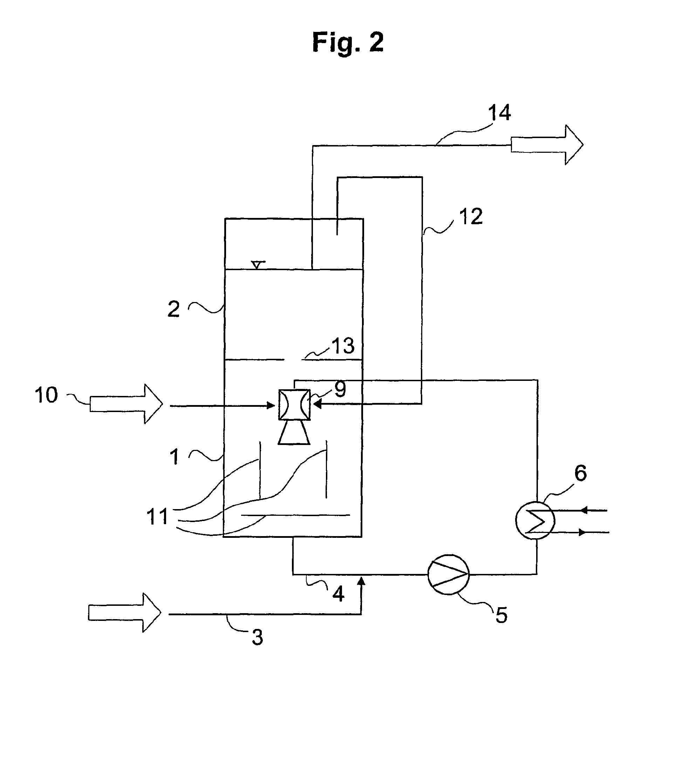

Vorteilhafte Ausgestaltungen des Hydrierungsschritts des erfindungsgemäßen Verfahrens im Hinblick auf eine kontinuierliche Reaktionsführung sind in den Figuren 1 und 2 dargestellt und werden im Folgenden erläutert.

Figur 1 zeigt die schematische Darstellung einer zur Durchführung des Hydrierverfahrens geeigneten zweistufigen Reaktorkaskade, wobei aus Gründen der Übersichtlichkeit auf die Wiedergabe solcher Details verzichtet ist, die für die Erläuterung der Erfindung nicht relevant sind. Die Anlage umfasst einen ersten Hydrierreaktor (1) und einen zweiten Hydrierreaktor (8). Der Hydrierreaktor (1) ist als Kreislaufreaktor und der Hydrierreaktor (8) als adiabatischer Strömungsrohrreaktor ausgeführt. Über die Rohrleitung (2) wird Wasserstoffgas unter Druck in den Reaktor (1) eingeleitet, und über die Rohrleitung (3) wird eine Lösung der zu hydrierenden Verbindung in den Reaktor (1) eingeleitet. Wenn der Katalysator nicht in der Edukt-Lösung enthalten ist, wird er über eine weitere Leitung (10) entweder direkt dem Reaktor oder vor die Kreislaufpumpe zugefahren. Über die Rohrleitung (4) und die Pumpe (5) wird ein Austrag aus dem Reaktor (1) entnommen, im Wärmetauscher (6) abgekühlt und in zwei Teilströme (7a) und (7b) geteilt. Der Teilstrom (7a) wird als Kreisstrom dem Reaktor (1) wieder zugeführt. Die charakteristische Verweilzeitverteilung im Reaktor (1) hängt im Wesentlichen vom umgepumpten Strom (7a) ab. Über die Rohrleitung (7b) wird der zweite Teilstrom zur Vervollständigung der Hydrierung dem Reaktor (8) zugeführt. Der Ausschleusstrom (4) kann gelöste oder gasförmige Anteile z. B. an Wasserstoff enthalten. In einer alternativen Ausführung wird der Strom (4) einem Phasentrennbehälter zugeführt und die gasförmigen Anteile über die separate Leitung (11) dem Reaktor (8) zugeführt. In einer weiteren alternativen Ausführung erfolgt die Beschickung des Reaktors (8) mit Wasserstoff nicht über einen dem Reaktor (1) entnommenen gasförmigen Feed, sondern mit frischem Wasserstoff über eine separate Zuleitung. Das Hydrierprodukt verlässt Reaktor (8) über die Rohrleitung (9).

Figur 2 zeigt die schematische Darstellung eines zur Durchführung des Hydrierverfahrens geeigneten Reaktors aus zwei Hydrierkompartimenten, wobei wiederum aus Gründen der Übersichtlichkeit auf die Wiedergabe solcher Details verzichtet ist, die für die Erläuterung der Erfindung nicht relevant sind. Der Reaktor umfasst zwei Hydrierkompartimente (1) und (2), die beide rückvermischt ausgeführt sind. Kompartiment (1) ist als Strahlschlaufenreaktor ausgeführt. Im Kompartiment (2) erfolgt die Hydrierung unter quasi-adiabatischen Bedingungen. Aus dem Kompartiment (1) wird über die Umwälzpumpe (5) ein Ausschleusstrom entnommen und gemeinsam mit zugeführtem Wasserstoffgas (3) über Wärmetauscher (6) der flussgeregelten Düse (9) zugeführt. Der Düse (9) kann im Bedarfsfall über Zuleitung (10) Wasserstoffgas zugeführt werden. Der Ausstoßstrom der Düse (9) wird durch die Prallbleche (11) begrenzt. Die Zufuhr des Nachreaktors (2) erfolgt über einen Lochboden mit mindestens einem Loch (13). Zur besseren Durchmischung kann ein Gaskreislauf (12) unter Einsatz eines Ejektors (9) eingesetzt werden. Das Hydrierprodukt wird dem Flüssigkeitsraum des Kompartiments (2) über Rohrleitung (14) entnommen.

Die Erfindung wird anhand der folgenden nicht einschränkenden Beispiele erläutert.

### Beispiele

### Beispiel 1:

Herstellung von

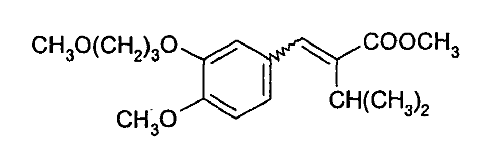

Zu einer Lösung von 88,5 g Diisopropylamin in 300 ml Tetrahydrofuran wurden bei -50°C 544 ml einer 15%igen Lösung von n-Butyllithium in Hexan, 98,2 g Methylisovalerat in 45 ml Tetrahydrofuran und 170 g 4-Methoxy-3-(3-methoxypropyloxy)-benzaldehyd in 75 ml Tetrahydrofuran zugetropft. Die resultierende Lösung ließ man innerhalb von 2 h auf Raumtemperatur erwärmen und rührte noch 1 h bei dieser Temperatur nach. Anschließend wurden tropfenweise 300 ml Wasser zu der Reaktionslösung gegeben, der pH-Wert mit konzentrierter HCl auf 1 eingestellt, die Phasen separiert und die wässrige Phase anschließend zweimal mit 300 ml Toluol extrahiert. Die organischen Phasen wurden vereinigt und das Lösungsmittel am Rotationsverdampfer abgedampft. Der Rückstand wurde in 500 ml Toluol aufgenommen, mit 6 g p-Toluolsulfonsäure versetzt und anschließend 3,5 h am Wasserabscheider unter Rückfluss erhitzt. Das Reaktionsgemisch wurde mit 150 ml gesättigter NaHCO₃-Lösung und 300 ml Wasser gewaschen, über Natriumsulfat getrocknet und das Lösungsmittel am Rotationsverdampfer abgezogen. Man erhielt 242 g Produkt.

Die Analyse des Reaktionsprodukts erfolgte mittels HPLC nach folgender Methode:
Säule: Waters Symmetry C18 5 µm, 250 x 4,6 mm
Eluent: A) 0,1 Vol-% H₃PO₄ in Wasser, B) 0,1 Vol% H₃PO₄ in CH₃CN
Gradient (bezogen auf Eluent B): 0 min (35 %) 20 min (100 %) 30 min (100 %) 32 min (35%)
Fluss: 1 ml/min, Temperatur: 20°C, Injektionsvolumen: 5 µl
Detektion: UV-Detektor bei 205 nm, BW = 4 nm

Bei dieser Methode eluierte der cis-Ester bei 15,7 min, der trans-Ester bei 16,2 min, die cis-Säure bei 10,6 min, die trans-Säure bei 10,9 min und der als Edukt eingesetzte aromatische Aldehyd bei 7,9 min.

Das erhaltene Produkt enthielt 69,1 % cis-Ester, 21,0 % trans-Ester, 0,8 % Aldehyd, restliche Komponenten nicht zugeordnet (Flächen-% der HPLC-Peaks).

Die Hydrolyse des erhaltenen Estergemischs kann nach üblichen Verfahren, beispielsweise mit KOH in einem Ethanol/Wasser-Gemisch erfolgen.

### Beispiel 2:

Herstellung von

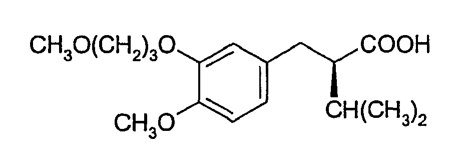

In einem 300 ml Stahlautoklaven wurden 30,1 g des nach Esterhydrolyse erhaltenen cis/trans-Säuregemischs in 55,4 g Methanol unter Schutzgasatmosphäre vorgelegt. Nach Zugabe von 2,05 mg (R)-Phanephos-Rh-(COD)BF₄ x 1 (C₂H₅)₂O wurde 12 h bei einem Wasserstoffdruck von 200 bar und einer Temperatur von 100 °C hydriert. Nach 12 h war die Hydrierung quantitativ. Der Enantiomerenüberschuss des Produkts betrug 83%.

Die Analytik sowohl des Produkts der Hydrierung als auch der anschließenden Kristallisation (Beispiele 3 und 4) erfolgte mittels HPLC nach der folgenden Methode:
Säule: CHIRALPAK AD-H (250 x 4,6 mm)
Eluent: Gemisch aus 950 ml n-Heptan, 50 ml Ethanol und 2 ml Trifluoressigsäure
Fluss: 1,0 ml/min, Säulentemperatur 25 °C, Injektionsvolumen 25 µl
Detektion: UV-Detektor bei 225 nm

Bei dieser Methode eluierte das cis-Isomer (Edukt) bei 22,3 min, das trans-Isomer (Edukt) bei 30,7 min, das (S)-Enantiomer (Produkt) bei 11,7 min und das (R)-Enantiomer (Produkt) bei 14,0 min.

### Beispiel 3:

### Enantiomerenanreicherung durch Kristallisation mit Ammoniak

95,6 g eines nach Beispiel 2 erhaltenen rohen Hydrierungsprodukts wurden in 750 ml Isopropanol gelöst und mit 44,2 ml 25%iger Ammoniaklösung unter Rühren versetzt.

Nach 10 min konnte eine Kristallbildung beobachtet werden. Anschließend ließ man noch 3 h bei Raumtemperatur nachrühren, kühlte die Kristalllösung auf -10 °C und isolierte die Kristalle durch Filtration. Der erhaltene Feststoff wurde zweimal mit 100 ml kaltem Petrolether gewaschen und über Nacht bei 30 °C im Trockenschrank getrocknet.

Man erhielt das Ammoniumsalz mit einer Ausbeute von 78 % bezogen auf das eingesetzte Rohprodukt mit einem ee-Wert von 98,9 %.

### Beispiel 4:

### Enantiomerenanreicherung durch Kristallisation mit LiOH

0,5 g eines nach Beispiel 2 erhaltenen rohen Hydrierungsprodukts wurden in 5 ml Isopropanol gelöst, mit 40 mg LiOH versetzt und die resultierende Suspension 1 h bei Raumtemperatur nachgerührt. Die resultierenden Kristalle wurden durch Filtration isoliert und der Feststoff zweimal mit 2 ml kaltem Petrolether gewaschen und über Nacht bei 30 °C im Trockenschrank getrocknet. Es wurden 0,3 g Kristalle (60 %) mit einem ee-Wert von 97,5 % erhalten.

### Beispiel 5:

### Herstellung von Synthon A-Säure

Das in Beispiel 3 erhaltene Ammoniumsalz wurde in 500 ml Wasser gelöst und der pH-Wert durch Zugabe von 30 ml konz. HCl auf einen Wert von 1 eingestellt. Die wässrige Phase wurde zweimal mit je 250 ml Toluol extrahiert, die vereinigten organischen Phasen mit vollentsalztem Wasser gewaschen und anschließend das Lösungsmittel am Rotationsverdampfer auf 150 ml eingeengt. Nach 10-minütigem Rühren bei Raumtemperatur wurde Kristallbildung beobachtet. Anschließend ließ man noch 3 h bei Raumtemperatur nachrühren, kühlte die Kristalllösung auf -10 °C ab und isolierte die Kristalle durch Filtration. Der resultierende Feststoff wurde zweimal mit je 100 ml kaltem Petrolether gewaschen und über Nacht bei 30 °C im Trockenschrank getrocknet. Man erhielt 69,3 g Synthon A-Säure als weißen Feststoff in einer Ausbeute von 99 % und mit einem ee-Wert von 98,9 %.

### Beispiel 6:

### "Scale-up" von Beispiel 1

In einem 1m³-Edelstahlkessel wurden 68,4 kg Diisopropylamin und 155 kg Tetrahydrofuran (THF) vorgelegt und auf -50 °C gekühlt. Dann wurden nacheinander 274 kg einer 15%igen Lösung von n-Butyllithium in Hexan, 72,7 kg Methylisovalerat, 30 kg THF, und 139 kg 4-Methoxy-3-(3-methoxypropyloxy)-benzaldehyd gefolgt von 30 kg THF zudosiert, wobei die Temperatur unterhalb von -30 °C gehalten wurde. Nach Zulaufende wurde der Reaktor mit 10 K/h auf 20 °C erwärmt. In einem 2,5 m³-Stahl/Emailkessel wurden 500 L vollentsalztes Wasser vorgelegt, der Inhalt des Edelstahlkessels bei 20 °C zugefahren und der Edelstahlkessel mit 88 kg THF nachgespült. Dann wurde der pH-Wert durch Zugabe von 200 kg 31 %iger HCl auf 1 eingestellt und die Phasen getrennt. Die organische obere Phase wurde in einem 1 m³-Stahl/Email-Kessel schrittweise auf 400 mbar evakuiert und das THF abdestilliert. Nach Zugabe von 585 kg Toluol und 5,4 kg p-Toluolsulfonsäure in 12 L vollentsalztem Wasser wurde vom Kesselinhalt Toluol/Wasser azeotrop abdestilliert, bis reines Toluol überging. Nach Abkühlen auf 20 °C wurde der Kesselinhalt mit 200 L gesättigter NaHCO₃-Lösun und 200 L Wasser gewaschen und die organische Phase direkt in Beispiel 7 eingesetzt. Die rohe 28%ige Produktlösung enthielt 160 kg cis-trans-Isomerengemisch (3.2:1).

### Beispiel 7:

### Hydrolyse der in Beispiel 6 dargestellten Verbindung

In einem 2 m³ Edelstahlkessel wurden die Produktlösungen aus zwei Partien der Vorstufe (Beispiel 6) vereinigt und bei einem Druck von 150 mbar das Toluol weitgehend abdestilliert. Bei einer Innentemperatur von 80 °C wurden 720 kg 25%ige NaOH zugefahren und 6 h bis zum Erreichen einer Innentemperatur von 115 °C destilliert. Der Kesselinhalt wurde auf 60°C abgekühlt und zur Phasentrennung absitzen gelassen. Nach Abtrennung von 500 L einer klaren wässrigen Phase wurden zu der braunen organischen Phase im Kessel 630 kg Wasser und 300 kg Toluol gegeben und bei 60 °C 30 Minuten gerührt. Anschließend wurden 1100 L einer wässrigen Phase abgelassen und die organische Phase verworfen. Die wässrige Phase wurde ein zweites Mal mit 300 kg Toluol extrahiert. Die wässrige Phase wurde dann in einem 2,5 m³-Stahl/EmailKessel mit 590 kg Toluol versetzt, durch Zugabe von 105 kg 75%iger Schwefelsäure angesäuert und 30 Minuten nachgerührt. Die Phasen wurden getrennt und die wässrige Phase erneut mit 590 kg Toluol extrahiert. Die organischen Phasen wurden vereinigt und mit 700 kg vollentsalztem Wasser gewaschen. Die gewaschene organische Phase wurde bei 150 mbar zum Sieden erhitzt und das Toluol abdestilliert, bis die Sumpftemperatur 120 °C betrug. Der verbliebene Sumpf wurde durch Zugabe von 350 kg Methanol verdünnt. Erhalten wurden 302 kg Säure als cis-trans-Isomerengemisch (3.2:1).

### Beispiel 8:

### "Scale-up" von Beispiel 2

In einem 3,5 m³ Stahlautoklaven wurden 486 kg des analog zu Beispiel 7 erhaltenen cis/trans-Säuregemischs in 1118 kg Methanol unter Schutzgasatmosphäre vorgelegt. Nach Zugabe einer methanolischen Lösung von 64,3 g (R)-Phanephos-Rh-(COD)BF₄ wurde bei einem Wasserstoffdruck von 200 bar und einer Temperatur von 75 °C hydriert. Nach 14 h war die Hydrierung quantitativ. Der Enantiomerenüberschuss des Produkts betrug 86 %.

### Beispiel 9:

### "Scale-up" der Beispiele 3 und 5

In einen 2 m³ Edelstahlkessel wurden 1000 kg einer 25%igen Lösung des Hydrierungsprodukts aus Beispiel 8 Stufe gefüllt und das Methanol bei einem Druck von 600 mbar weitgehend abdestilliert. Zum verbliebenen Sumpf wurden 1000 kg Isopropanol gegeben und bei 50 °C 57 kg einer 25%igen wässrigen Ammoniaklösung zugegeben. Nach Zulaufende wurde 30 Minuten bei 50°C nachgerührt, anschließend mit 10 K/h auf 0 °C abgekühlt und 1 h bei 0 °C nachgerührt. Die Kristallmaische wurde in 4 Portionen in einer Schälzentrifuge zentrifugiert, die Kristalle mit jeweils 100 kg Isopropanol nachgewaschen und mit einer Restfeuchte von ca. 60 % ausgetragen.

Die Kristalle wurden in einem 2m³ Stahl-Emailkessel in 800 kg Wasser gelöst und mit 400 kg Toluol überschichtet. Bei 30 °C wurden 120 L einer 31%igen HCl-Lösung zugegeben und 30 Minuten nachgerührt. Nach Phasentrennung wurde die wässrige Phase erneut mit 400 kg Toluol extrahiert, die organischen Phasen vereinigt und mit 300 kg vollentsalztem Wasser gewaschen. Bei Normaldruck wurden 500 L Toluol abdestilliert. Erhalten wurden in einer 28%igen toluolischen Lösung 205 kg SynthonA-Säure mit einem ee von 99,2 %.

### Beispiel 10:

Herstellung von 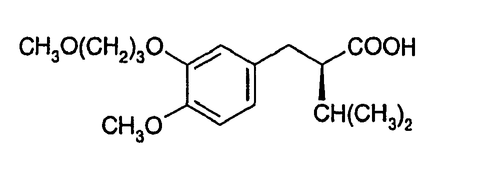 durch Hydrierung mit Phanephos bei 80 bar
In einem 300 ml Stahlautoklaven wurden 30 g des nach Esterhydrolyse gemäß Beispiel 1 erhaltenen cis/trans-Säuregemischs in 59 g Methanol unter Schutzgasatmosphäre vorgelegt. Nach Zugabe von 3,8 mg (R)-Phanephos-Rh-(COD)BF₄ wurde bei einem Wasserstoffdruck von 80 bar und einer Temperatur von 90°C hydriert. Nach 20 h war die Hydrierung quantitativ. Der Enantiomerenüberschuss des Produkts betrug 83%.

### Beispiel 11:

Herstellung von 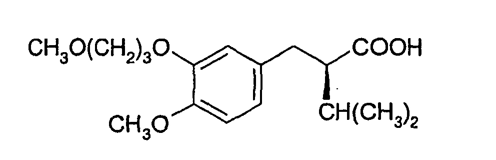 durch Hydrierung mit (Ligand C) 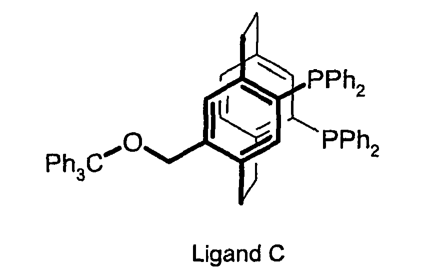 In einem 300 ml Stahlautoklaven wurden 30 g des nach Esterhydrolyse analog Beispiel 1 erhaltenen cis/trans-Säuregemischs in 62 g Methanol unter Schutzgasatmosphäre vorgelegt. Nach Zugabe von 5,0 mg (R)-(Ligand C)-Rh-(NBD)BF₄ wurde bei einem Wasserstoffdruck von 80 bar und einer Temperatur von 90°C hydriert. Nach 20 h war die Hydrierung quantitativ. Der Enantiomerenüberschuss des Produkts betrug 83 %.

### Beispiel 12:

Herstellung von 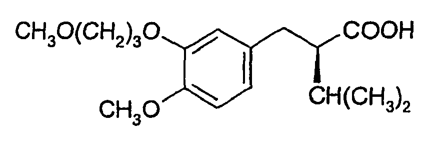 durch Hydrierung mit Ligand D

In einem 300 ml Stahlautoklaven wurden 30 g des nach Esterhydrolyse analog Beispiel 1 erhaltenen cis/trans-Säuregemischs in 60 g Methanol unter Schutzgasatmosphäre vorgelegt. Nach Zugabe von 4,7 mg (Ligand D)-Rh-(COD)BF₄ wurde bei einem Wasserstoffdruck von 200 bar und einer Temperatur von 100 °C hydriert. Nach 8 h war die Hydrierung quantitativ. Der Enantiomerenüberschuss des Produkts betrug 83 %.

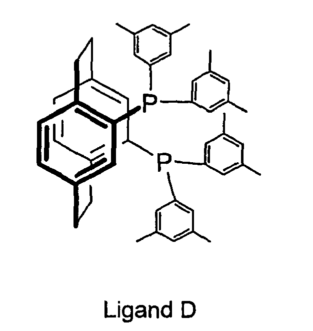

### Beispiel 13:

Herstellung von 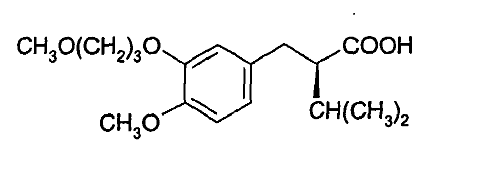 durch Hydrierung mit Ligand E

In einem 300 ml Stahlautoklaven wurden 40 g des nach Esterhydrolyse analog Beispiel 1 erhaltenen cis/trans-Säuregemischs in 40 g Methanol unter Schutzgasatmosphäre vorgelegt. Nach Zugabe von 6,0 mg (Ligand E)-Rh-(COD)BF₄ wurde bei einem Wasserstoffdruck von 80 bar und einer Temperatur von 90 °C hydriert. Nach 12 h war die Hydrierung quantitativ. Der Enantiomerenüberschuss des Produkts betrug 80 %.

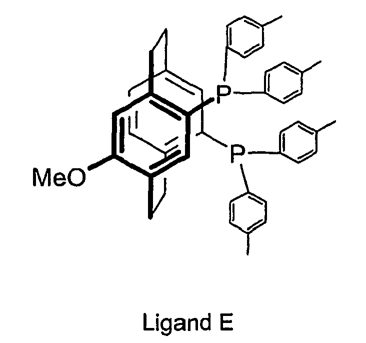

### Beispiel 14:

Herstellung von 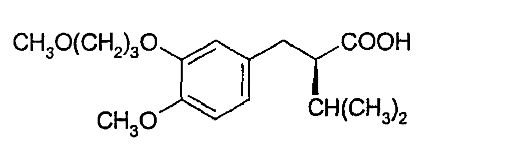 durch Hydrierung mit Ligand F

In einem 300 ml Stahlautoklaven wurden 40 g des nach Esterhydrolyse analog Beispiel 1 erhaltenen cis/trans-Säuregemischs in 40 g Methanol unter Schutzgasatmosphäre vorgelegt. Nach Zugabe von 5,9 mg (Ligand F)-Rh-(COD)BF₄ (als methanolische Lösung) wurde bei einem Wasserstoffdruck von 80 bar und einer Temperatur von 90 °C hydriert. Nach 12 h war die Hydrierung quantitativ. Der Enantiomerenüberschuss des Produkts betrug 82 %.

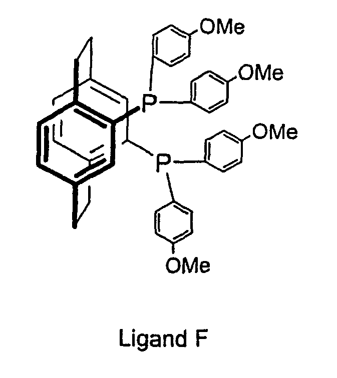

### Beispiel 15:

Herstellung von 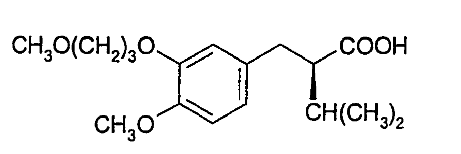 durch Hydrierung mit Ligand G

In einem 300 ml Stahlautoklaven wurden 40 g des nach Esterhydrolyse analog Beispiel 1 erhaltenen cis/trans-Säuregemischs in 40 g Methanol unter Schutzgasatmosphäre vorgelegt. Nach Zugabe von 5,3 mg (Ligand G)-Rh-(COD)BF₄ wurde bei einem Wasserstoffdruck von 80 bar und einer Temperatur von 90 °C hydriert. Nach 16 h war die Hydrierung quantitativ. Der Enantiomerenüberschuss des Produkts betrug 81 %.

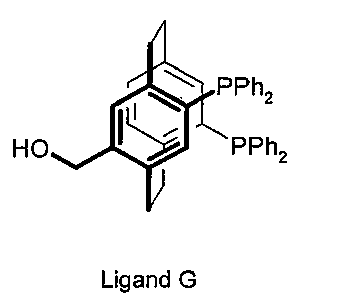

### Beispiel 16:

Herstellung von 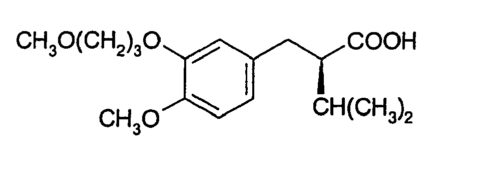 durch Hydrierung mit Ligand H

In einem 300 ml Stahlautoklaven wurden 40 g des nach Esterhydrolyse analog Beispiel 1 erhaltenen cis/trans-Säuregemischs in 40 g Methanol unter Schutzgasatmosphäre vorgelegt. Nach Zugabe von 5,5 mg (Ligand H)-Rh-(COD)BF₄ wurde bei einem Wasserstoffdruck von 80 bar und einer Temperatur von 90 °C hydriert. Nach 16 h war die Hydrierung quantitativ. Der Enantiomerenüberschuss des Produkts betrug 82 %.

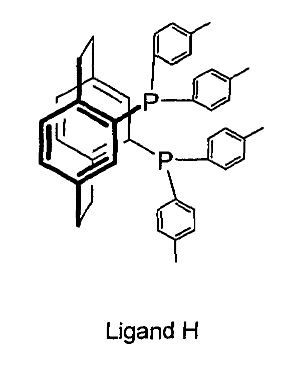

### Beispiel 17:

### Umkristallisation von rohem Synthon A

In einem 1 m³ Stahlemailkessel wurden 200 kg eines Synthon A-Rohprodukts mit einem Gehalt von 89,1 Gew.% (bestimmt per HPLC) und einem ee von 97,2 % bei 50 °C mit 400 kg Methanol versetzt und auf 30 °C abgekühlt. Nach Animpfen mit Kristallen von reinem Synthon A wurde mit einer Rate von 10 K/h auf -10 °C abgekühlt und die resultierende Kristallmaische über einen Prozessfilter abfiltriert, mit etwa 100 kg kaltem Methanol gewaschen und im Vakuum getrocknet. Man erhielt 144 kg Synthon A als weiße Kristalle mit einem Gehalt von 99,5 Gew.-%. Der Enantiomerenüberschuss betrug 99,8 %.

## Patentansprüche

1. Verfahren zur Herstellung von optisch aktiven Verbindungen der allgemeinen Formel I 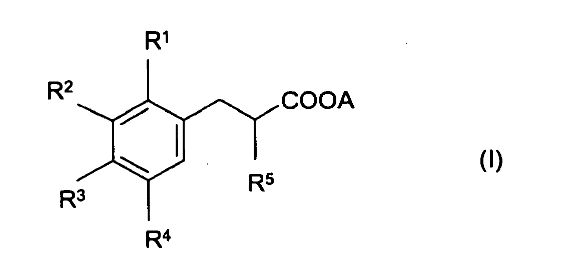 worin
R¹, R², R³ und R⁴ unabhängig voneinander für Wasserstoff, C₁-C₆-Alkyl, Halogen-C₁-C₆-alkyl, Hydroxy-C₁-C₆-alkyl, C₁-C₆-Alkoxy, Hydroxy-C₁-C₆-alkoxy, C₁-C₆-Alkoxy-C₁-C₆-alkyl, Hydroxy-C₁-C₆-alkoxy-C₁-C₆-alkyl, C₁-C₆-Alkoxy-C₁-C₆-alkoxy oder Hydroxy-C₁-C₆-alkoxy-C₁-C₆-alkoxy stehen,
R⁵ für C₁-C₆-Alkyl, C₅-C₈-Cycloalkyl, Phenyl oder Benzyl steht, und
A für Wasserstoff oder ein Kationäquivalent steht, bei dem man
- das cis-Isomer oder ein cis/trans-Isomerengemisch von Verbindungen der allgemeinen Formel II 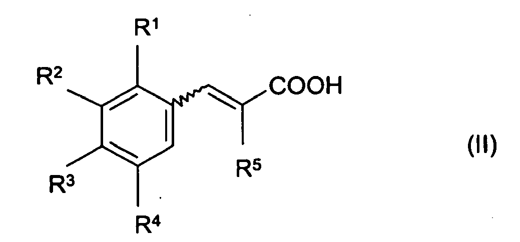
worin R¹ bis R⁵ die zuvor angegebenen Bedeutungen besitzen, einer enantioselektiven Hydrierung in Gegenwart eines chiralen Hydrierungskatalysators unterzieht wobei zur Hydrierung ein Übergangsmetallkomplex als Katalysator eingesetzt wird, der als Liganden wenigstens eine Verbindung der Formel 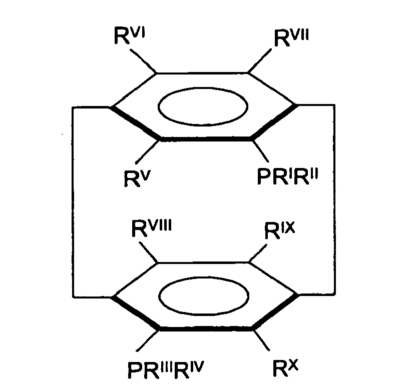 umfasst, worin
R^{I}, R^{II}, R^{III} und R^{IV} unabhängig voneinander für Alkyl, Cycloalkyl, Heterocycloalkyl, Aryl oder Hetaryl stehen, und
R^{V}, R^{VI}, R^{VII}, R^{VIII}, R^{IX} und R^{X} unabhängig voneinander für Wasserstoff, Alkyl, Alkylen-OH, Alkylen-NE¹E², Alkylen-SH, Alkylen-OSiE³E⁴, Cycloalkyl, Heterocycloalkyl, Aryl, Hetaryl, OH, SH, Polyalkylenoxid, Polyalkylenimin, Alkoxy, Halogen, COOH, Carboxylat, SO₃H, Sulfonat, NE¹E², Nitro, Alkoxycarbonyl, Acyl oder Cyano stehen, worin E¹, E², E³ und E⁴ jeweils gleiche oder verschiedene Reste, ausgewählt unter Wasserstoff, Alkyl, Cycloalkyl, Aryl und Alkylaryl bedeuten,
unter Erhalt eines an einem Enantiomeren angereicherten Enantiomerengemischs,
- das bei der Hydrierung erhaltene Enantiomerengemisch zur weiteren Enantiomerenanreicherung einer Kristallisation durch Zugabe eines basischen Salzbildners in einem Lösungsmittel unterzieht und den dabei gebildeten, bezüglich eines Stereoisomers angereicherten Feststoff isoliert, und
- gegebenenfalls das isolierte Isomer einer Protonierung oder einem Kationenaustausch unter Erhalt der optisch aktiven Verbindung der Formel I unterzieht.

2. Verfahren nach Anspruch 1, wobei zur Hydrierung ein cis/trans-Isomerengemisch eingesetzt wird, das wenigstens 50 Gew.%, bevorzugt wenigstens 60 Gew.-%, insbesondere wenigstens 70 Gew.-% des cis-Isomers enthält.

3. Verfahren nach einem der vorhergehenden Ansprüche, wobei zur Hydrierung ein cis/trans-Isomerengemisch eingesetzt wird, das wenigstens 1 Gew.-%, bevorzugt wenigstens 5 Gew.-%, insbesondere wenigstens 10 Gew.-% des trans-Isomers enthält.

4. Verfahren nach Anspruch 1, wobei R^{I}, R^{II}, R^{III} und R^{IV} unabhängig voneinander für Phenyl, Tolyl, Methoxyphenyl, Xylyl oder Methoxyxylyl stehen.

5. Verfahren nach einem der Ansprüche 1 bis 4, wobei einer der Reste R^{V}, R^{VI} und R^{VII} und/oder einer der Reste R^{VIII}, R^{IX} und R^{X} für einen von Wasserstoff verschiedenen Rest stehen und der/die von Wasserstoff verschiedene(n) Rest(e) ausgewählt ist/sind unter C₁-C₆-Alkyl, C₁-C₄-Alkylen-OH, C₁-C₄-Alkylen-OSi(C₁-C₄-Alkyl)₂, C₁-C₄-Alkoxy, C₁-C₄-Alkylen-OC(Alkyl)₃ und C₁-C₄-Alkylen-OC(Aryl)₃.

6. Verfahren nach einem der Ansprüche 1 bis 5, wobei der Katalysator wenigstens einen Liganden aufweist, der ausgewählt ist unter Verbindungen der Formeln

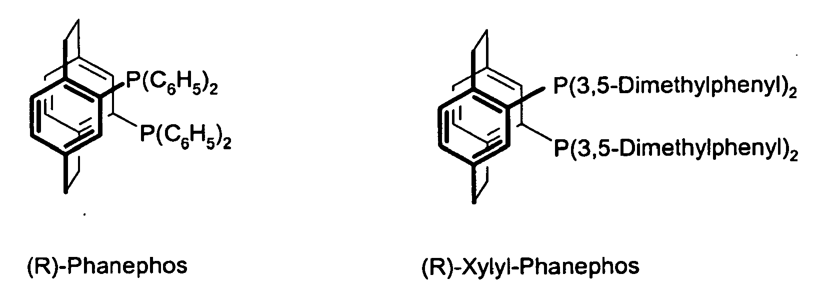

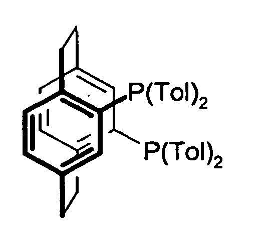

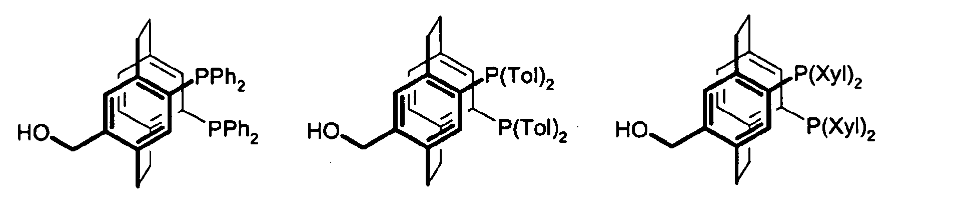

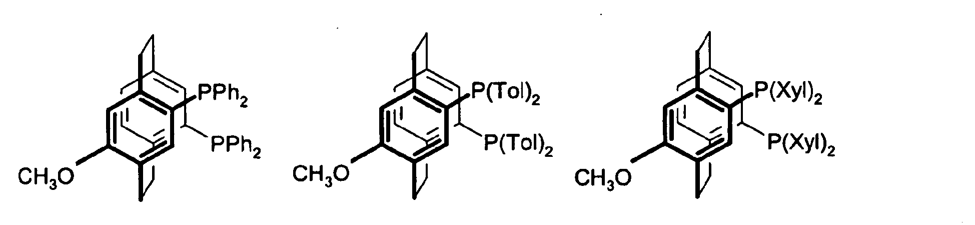

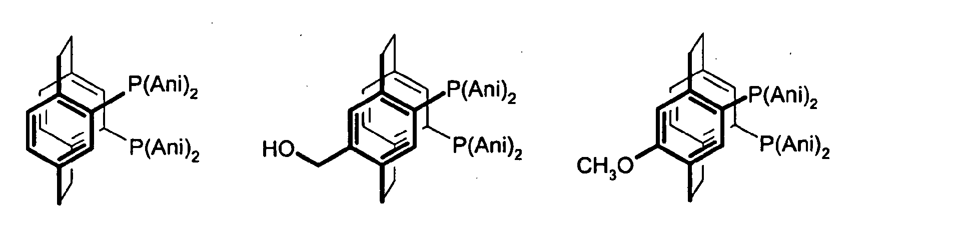

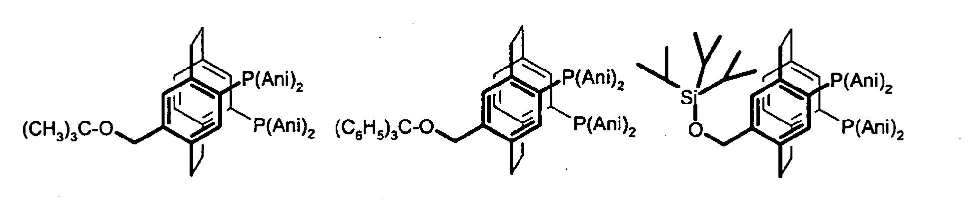

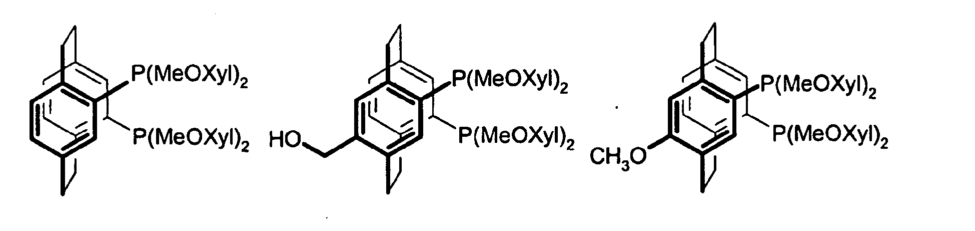

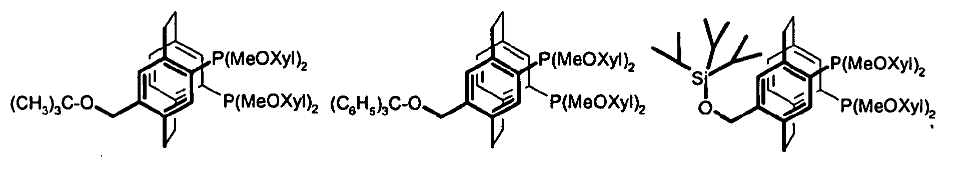

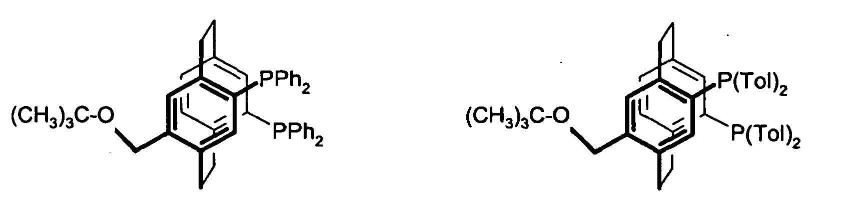

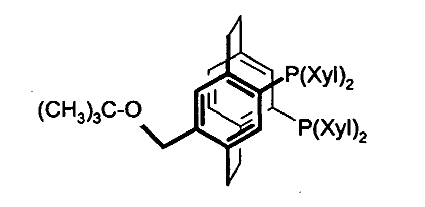

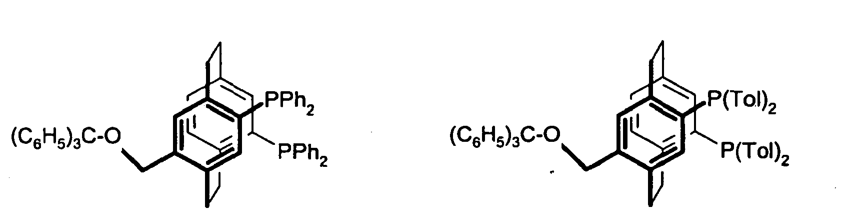

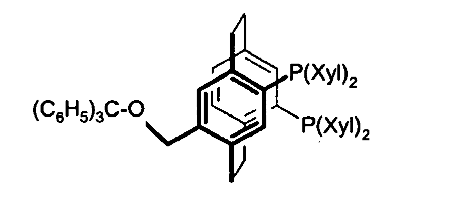

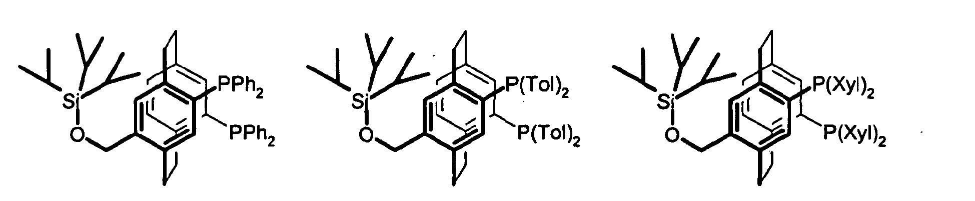

7. Verfahren nach einem der Ansprüche 1 bis 6, wobei der Katalysator wenigstens einen Liganden aufweist, der ausgewählt ist unter Verbindungen der Formeln

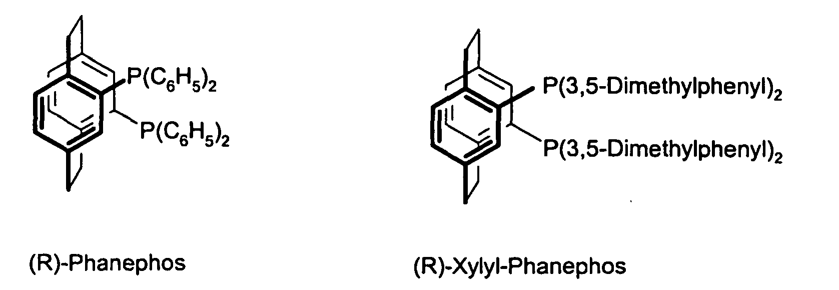

8. Verfahren nach einem der vorhergehenden Ansprüche, wobei die Hydrierung kontinuierlich erfolgt.

9. Verfahren nach Anspruch 8, bei dem man
i) in eine erste Reaktionszone ein Isomerengemisch von Verbindungen der allgemeinen Formel II und Wasserstoff einspeist und in Gegenwart eines chiralen Hydrierungskatalysators bis zu einem Teilumsatz umsetzt,
ii) aus der ersten Reaktionszone einen Strom entnimmt und in wenigstens einer weiteren Reaktionszone hydriert.

10. Verfahren nach einem der vorhergehenden. Ansprüche, wobei der zur Kristallisation eingesetzte Salzbildner ausgewählt ist unter achiralen basischen Verbindungen.

11. Verfahren nach Anspruch 10, wobei der Salzbildner ausgewählt ist unter Ammoniak, primären Aminen, Alkalihydroxiden und Erdalkalihydroxiden.

12. Verfahren nach einem der Ansprüche 10 oder 11, wobei zur Kristallisation Ammoniak oder LiOH als Salzbildner und Isopropanol als Lösungsmittel eingesetzt wird.

13. Verfahren nach einem der vorhergehenden Ansprüche, wobei der nach der Kristallisation isolierte Feststoff einen ee-Wert von mindestens 98 % aufweist.

14. Verfahren nach einem der vorhergehenden Ansprüche, wobei man eine optisch aktive Verbindung der Formel I mit folgender absoluter Konfiguration erhält 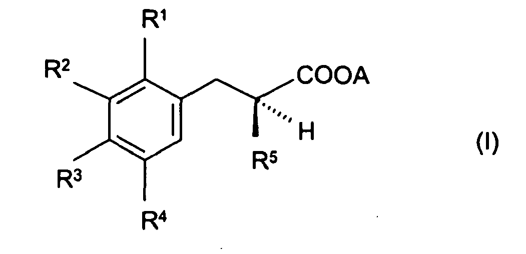 wobei R¹ bis R⁵ und A die in Anspruch 1 angegebenen Bedeutungen besitzen.

15. Verfahren zur Herstellung von optisch aktiven Verbindungen der allgemeinen Formel III 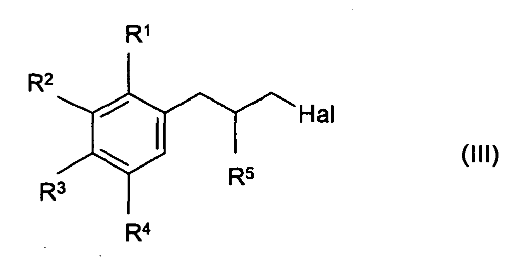 worin R¹ bis R⁵ die in Anspruch 1 angegebenen Bedeutungen besitzen und Hal für Cl, Br oder I steht, bei dem man
a) einen aromatischen Aldehyd der allgemeinen Formel V 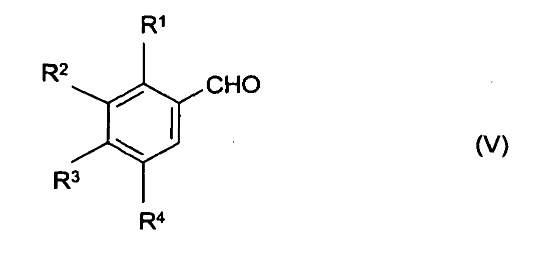 worin R¹ bis R⁴ die in Anspruch 1 angegebenen Bedeutungen besitzen, mit einem Carbonsäureester der allgemeinen Formel VI
R⁵-CH₂-COOR⁷ (VI)
worin R⁵ die in Anspruch 1 angegebenen Bedeutungen besitzt und R⁷ für Alkyl, Cycloalkyl, Aryl oder Alkylaryl steht, unter Erhalt von Verbindungen der allgemeinen Formel VII, 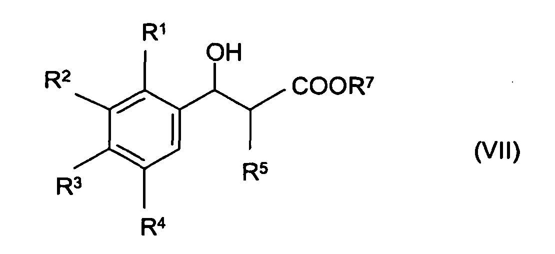 umsetzt,
b) in den Verbindungen der Formel VII die Hydroxylgruppe in eine bessere Abgangsgruppe überführt und einer Eliminierung unter Erhalt von Verbindungen der allgemeinen Formel VIII 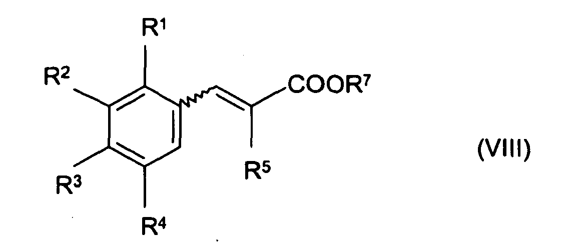 unterzieht,
c) die Verbindungen der Formel VIII einer Esterhydrolyse unter Erhalt von Verbindungen der allgemeinen Formel II 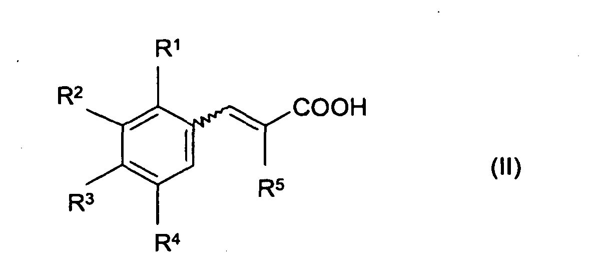 unterzieht,
d) die Verbindungen der Formel II einer enantioselektiven Hydrierung in Gegenwart eines chiralen Hydrierungskatalysators, wie in einem der Ansprüche 1 oder 4 bis 7 definiert, unterzieht, unter Erhalt eines an einem Enantiomeren angereicherten Enantiomerengemischs,
e) das bei der Hydrierung in Schritt d) erhaltene Enantiomerengemisch zur weiteren Enantiomerenanreicherung einer Kristallisation durch Zugabe eines basischen Salzbildners in einem Lösungsmittel unterzieht und den dabei gebildeten, bezüglich eines Stereoisomers angereicherten Feststoff isoliert,
f) gegebenenfalls das in Schritt e) isolierte Isomer einer Protonierung oder einem Kationenaustausch unter Erhalt der optisch aktiven Verbindung der Formel I unterzieht,
g) für den Fall, dass in der Verbindung der Formel I der Rest A für ein von Wasserstoff und Metallkationen verschiedenes Kationäquivalent steht, diesen einer Protonierung unterzieht,
h) die Säure oder das Metallsalz davon einer Reduktion unter Erhalt eines Alkohols der allgemeinen Formel IV 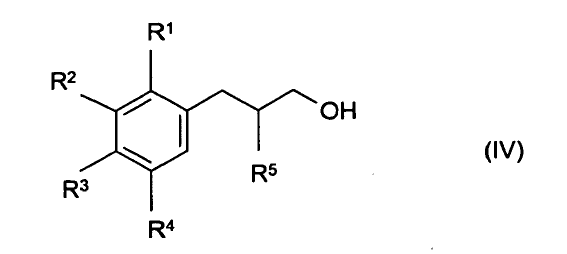 unterzieht, und
i) den Alkohol der Formel IV einer Halodehydroxylierung unter Erhalt der optisch aktiven Verbindung der Formel III 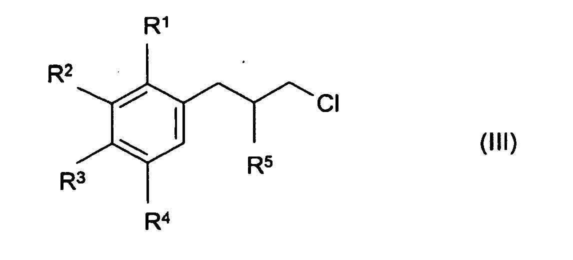 unterzieht.

16. Optisch aktive Verbindung der allgemeinen Formel I 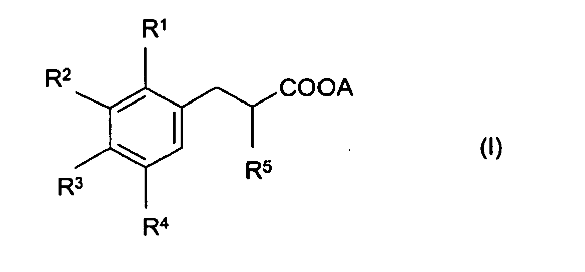 worin R¹ bis R⁵ die in Anspruch 1 angegebene Bedeutung besitzen und A für ein von Ammoniak, primären Aminen, Alkalimetallen und Erdalkalimetallen abgeleitetes Kation stehen.

17. Verbindung nach Anspruch 16 wobei R⁵ für einen verzweigten C₃-C₈-Alkylrest, insbesondere Isopropyl, steht.

18. Verbindung nach einem der Ansprüche 16 oder 17 der Formel

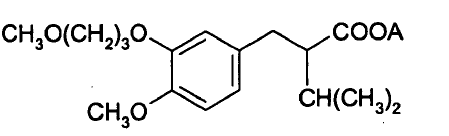

19. Verbindung nach einem der Ansprüche 16 bis 18, wobei A für NH₄⁺ oder Li⁺ steht.

## Claims

1. A method for preparing optically active compounds of the general formula I 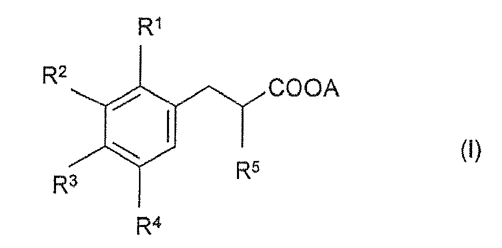 in which
R¹, R², R³ and R⁴ are independently of one another hydrogen, C₁-C₆-alkyl, halo-C₁-C₆-alkyl, hydroxy-C₁-C₆-alkyl, C₁-C₆-alkoxy, hydroxy-C₁-C₆-alkoxy, C₁-C₆-alkoxy-C₁-C₆-alkyl, hydroxy-C₁-C₆-alkoxy-C₁-C₆-alkyl, C₁-C₆-alkoxy-C₁-C₆-alkoxy or hydroxy-C₁-C₆-alkoxy-C₁-C₆-alkoxy,
R⁵ is C₁-C₆-alkyl, C₅-C₈-cycloalkyl, phenyl or benzyl, and
A is hydrogen or a cation equivalent, in which
- the cis isomer or a cis/trans isomer mixture of compounds of the general formula II 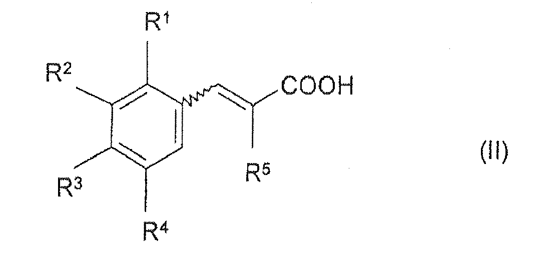
in which R¹ to R⁵ have the aforementioned meanings, is subjected to an enantioselective hydrogenation in the presence of a chiral hydrogenation catalyst,
where the catalyst employed for the hydrogenation is a transition metal complex which comprises as ligand at least one compound of the formula 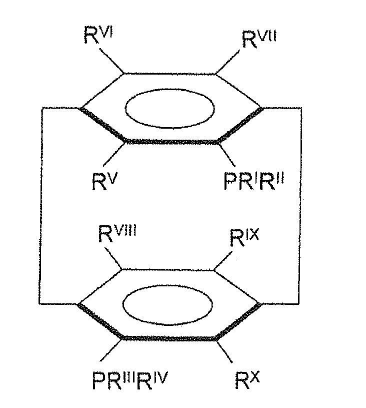 in which
R^{I}, R^{II}, R^{III} and R^{IV} are independently of one another alkyl, cycloalkyl, heterocycloalkyl, aryl or hetaryl, and
R^{V}, R^{VI}, R^{VII}, R^{VIII}, R^{IX} and R^{X} are independently of one another hydrogen, alkyl, alkylene-OH, alkylene-NE¹E², alkylene-SH, alkylene-OSiE³E⁴, cycloalkyl, heterocycloalkyl, aryl, hetaryl, OH, SH, polyalkylene oxide, polyalkyleneimine, alkoxy, halogen, COOH, carboxylate, SO₃H, sulfonate, NE¹E², nitro, alkoxycarbonyl, acyl or cyano, in which E¹, E², E³ and E⁴ are each identical or different radicals selected from hydrogen, alkyl, cycloalkyl, aryl and alkylaryl,
to obtain a mixture of enantiomers enriched in one enantiomer,
- the mixture of enantiomers obtained in the hydrogenation is subjected, for further enantiomer enrichment, to a crystallization by adding a basic salt former in a solvent, and the solid which is formed thereby and is enriched in one stereoisomer is isolated, and
- if appropriate the isolated isomer is subjected to a protonation or a cation exchange to obtain the optically active compound of the formula I.

2. The method according to claim 1, where a cis/trans isomer mixture which comprises at least 50% by weight, preferably at least 60% by weight, in particular at least 70% by weight of the cis isomer is employed for the hydrogenation.

3. The method according to either of the preceding claims, where a cis/trans isomer mixture which comprises at least 1% by weight, preferably at least 5% by weight, in particular at least 10% by weight of the trans isomer is employed for the hydrogenation.

4. The method according to claim 1, where R^{I}, R^{II}, R^{III} and R^{IV} are independently of one another phenyl, tolyl, methoxyphenyl, xylyl or methoxyxylyl.

5. The method according to any of claims 1 or 4, where one of the radicals R^{V} R^{VI} and R^{VII} and/or one of the radicals R^{VIII}, R^{IX} and R^{X} are a radical different from hydrogen, and the radical(s) different from hydrogen is/are selected from C₁-C₆-alkyl, C₁-C₄-alkylene-OH, C₁-C₄-alkylene-OSi(C₁-C₄-alkyl)₂, C₁-C₄-alkoxy, C₁-C₄-alkylene-OC(alkyl)₃ and C₁-C₄-alkylene-OC(aryl)₃.

6. The method according to any of claims 1 to 5, where the catalyst has at least one ligand which is selected from compounds of the formulae

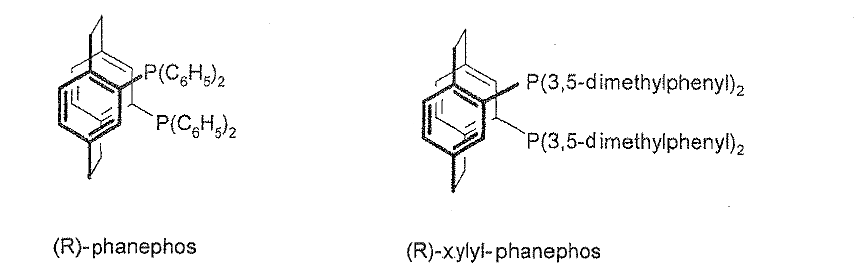

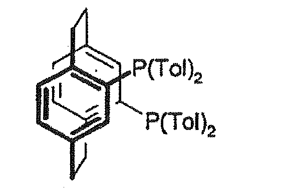

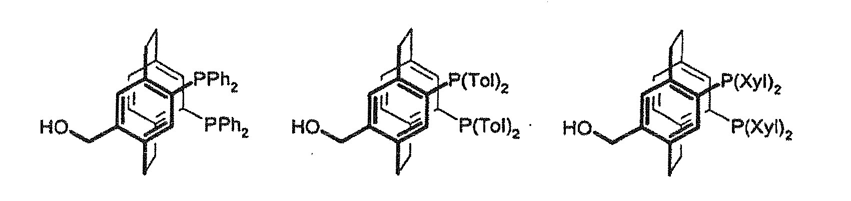

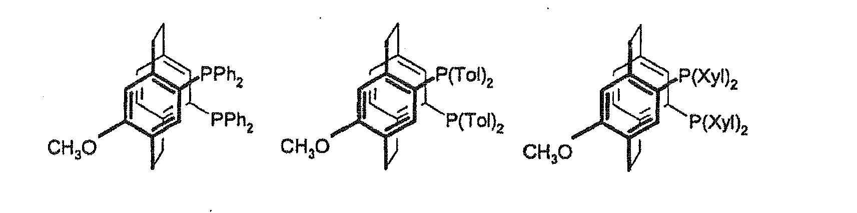

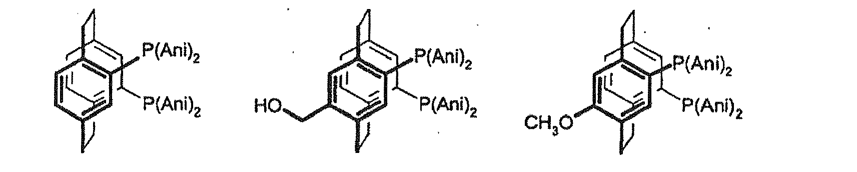

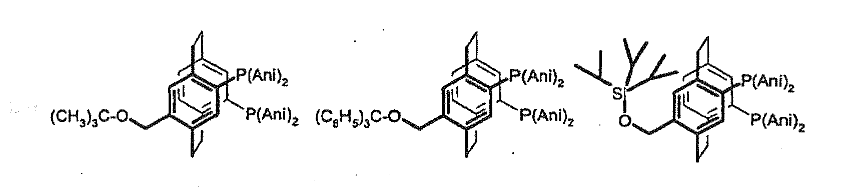

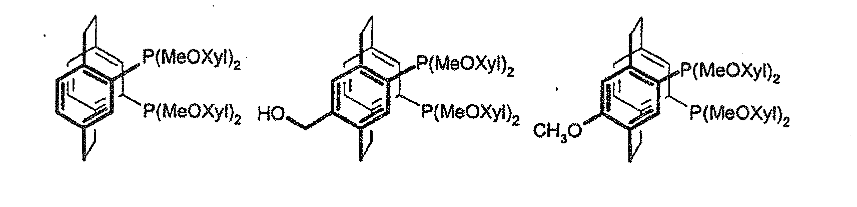

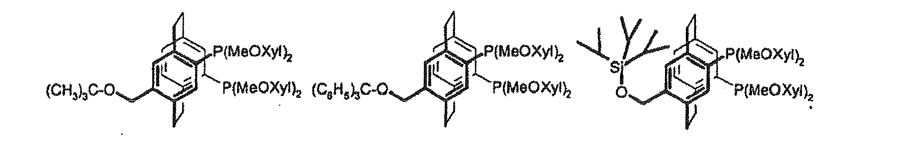

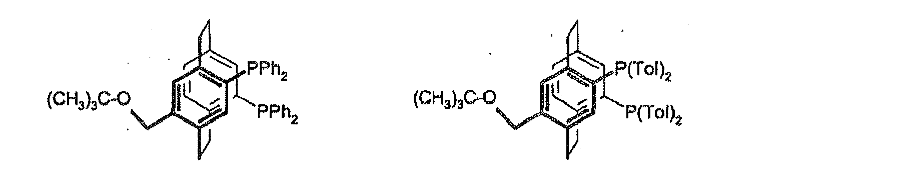

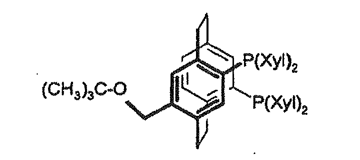

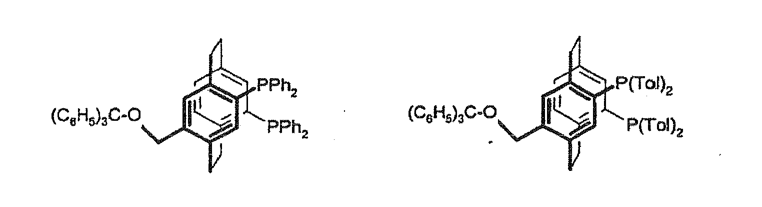

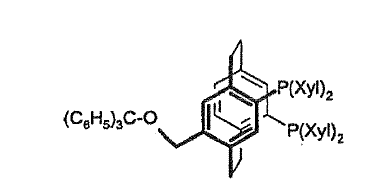

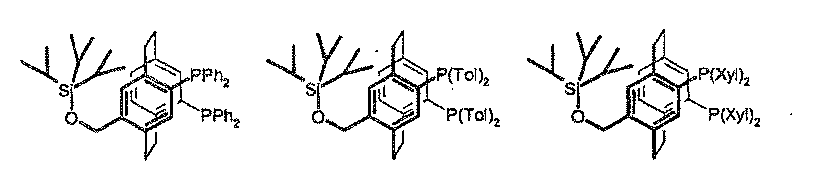

7. The method according to any of claims 1 to 6, where the catalyst has at least one ligand which is selected from compounds of the formulae

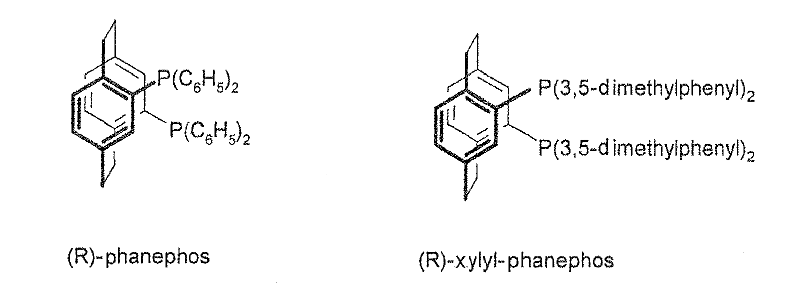

8. The method according to any of the preceding clams, where the hydrogenation takes place continuously.

9. The method according to claim 8, in which
i) a mixture of isomers of compounds of the general formula II and hydrogen are fed into a first reaction zone and reacted in the presence of a chiral hydrogenation catalyst as far as partial conversion,
ii) a stream is taken from the first reaction zone and hydrogenated in at least one further reaction zone.

10. The method according to any of the preceding claims, where the salt former employed for the crystallization is selected from achiral basic compounds.

11. The method according to claim 10, where the salt former is selected from ammonia, primary amines, alkali metal hydroxides and alkaline earth metal hydroxides.

12. The method according to either of claims 10 or 11, where ammonia or LiOH is employed as salt former and isopropanol is employed as solvent for the crystallization.

13. The method according to any of the preceding claims, where the solid isolated after the crystallization has an ee of at least 98%.

14. The method according to any of the preceding claims, where an optically active compound of the formula I with the following absolute configuration is obtained: 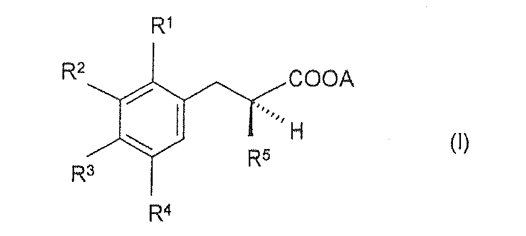 where R¹ to R⁵ and A have the meanings indicated in claim 1.

15. A method for preparing optically active compounds of the general formula III 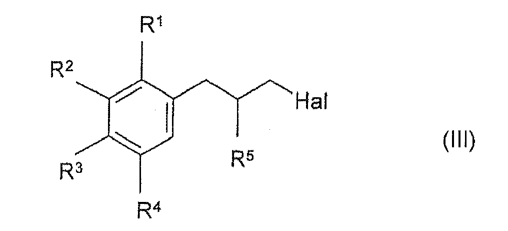 in which R¹ to R⁵ have the meanings indicated in claim 1, and Hal is Cl, Br or I, in which
a) an aromatic aldehyde of the general formula V 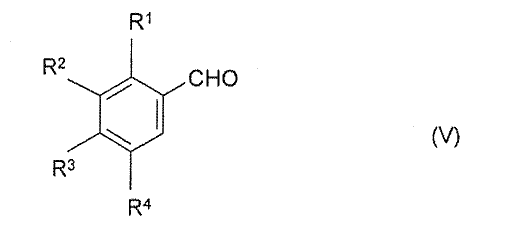 in which R¹ to R⁴ have the meanings indicated in claim 1, is reacted with a carboxylic ester of the general formula VI
R⁵-CH₂-COOR⁷ (VI)
in which R⁵ has the meanings indicated in claim 1, and R⁷ is alkyl, cycloalkyl, aryl or alkylaryl, to obtain compounds of the general formula VII 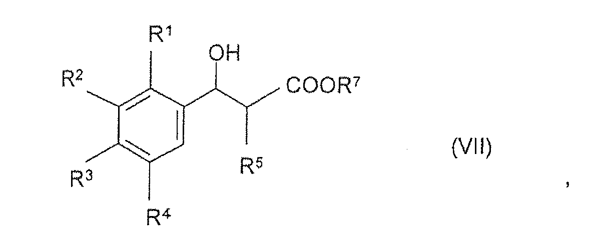
b) the hydroxyl group in the compounds of the formula VII is converted into a better leaving group and subjected to an elimination to obtain compounds of the general formula VIII 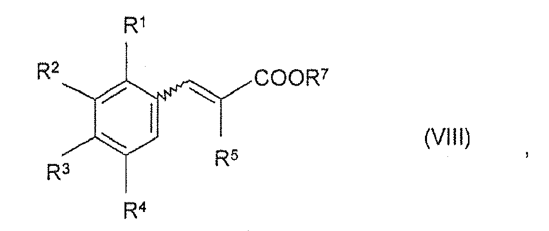
c) the compounds of the formula VIII are subjected to an ester hydrolysis to obtain compounds of the general formula II 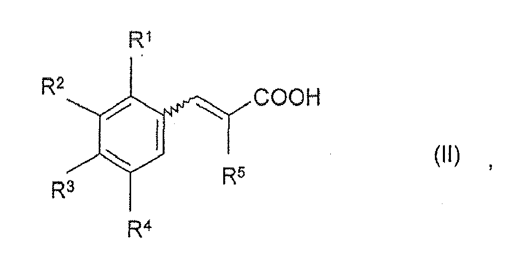
d) the compounds of the formula II are subjected to an enantioselective hydrogenation in the presence of a chiral hydrogenation catalyst, as defined in any of claims 1 or 4 to 7, to obtain a mixture of enantiomers enriched in one enantiomer,
e) the mixture of enantiomers obtained in the hydrogenation in step d) is subjected, for further enantiomer enrichment, to a crystallization by adding a basic salt former in a solvent, and the solid which is formed thereby and is enriched in one stereoisomer is isolated,
f) if appropriate the isomer isolated in step e) is subjected to a protonation or a cation exchange to obtain the optically active compound of the formula I,
g) in the case where the radical A in the compound of the formula I is a cation equivalent different from hydrogen and metal cations, this equivalent is subjected to a protonation,
h) the acid or the metal salt thereof is subjected to a reduction to obtain an alcohol of the general formula IV 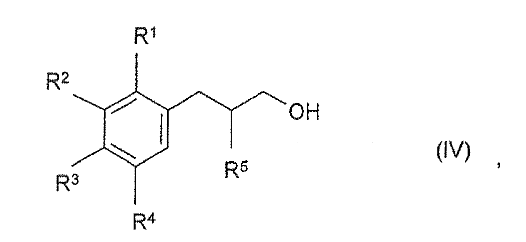 and
i) the alcohol of the formula IV is subjected to a halodehydroxylation to obtain the optically active compound of the formula III

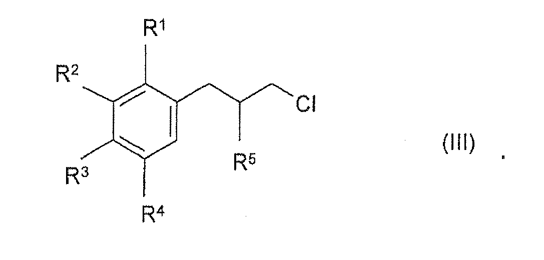

16. An optically active compound of the general formula I 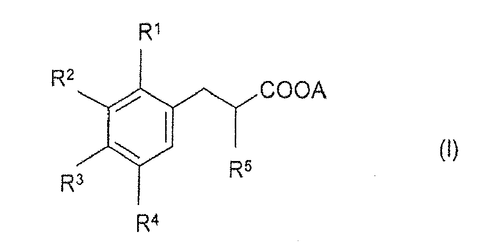 in which R¹ to R⁵ have the meaning indicated in claim 1, and A is a cation derived from ammonia, primary amines, alkali metals and alkaline earth metals.

17. The compound according to claim 16, where R⁵ is a branched C₃-C₈-alkyl radical, in particular isopropyl.

18. The compound according to either of claims 16 or 17 of the formula

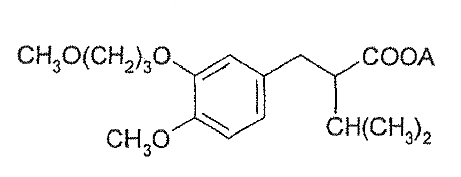

19. The compound according to any of claims 16 to 18, where A is NH₄⁺ or Li⁺.

## Revendications

1. Procédé de fabrication de composés optiquement actifs de formule générale I 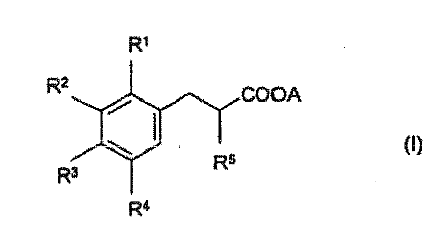 dans laquelle
R¹, R², R³ et R⁴ représentent indépendamment les uns des autres hydrogène, C₁-C₆-alkyle, halogène-C₁-C₆-alkyle, hydroxy-C₁-C₆-alkyle, C₁-C₆-alcoxy, hydroxy-C₁-C₆-alcoxy, C₁-C₆-alcoxy-C₁-C₆-alkyle, hydroxy-C₁-C₆-alcoxy-C₁-C₆-alkyle, C₁-C₆-alcoxy-C₁-C₆-alcoxy ou hydroxy-C₁-C₆-alcoxy-C₁-C₆-alcoxy,
R⁵ représente C₁-C₆-alkyle, C₅-C₈-cycloalkyle, phényle ou benzyle et
A représente hydrogène ou un équivalent cationique, selon lequel
- l'isomère cis ou un mélange d'isomères cis/trans de composés de formule générale II 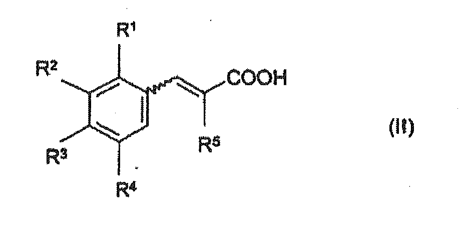
dans laquelle R¹ à R⁵ ont les significations données précédemment, est soumis à une hydrogénation énantiosélective en présence d'un catalyseur d'hydrogénation chiral, un complexe de métal de transition qui comporte en tant que ligands au moins un composé de formule 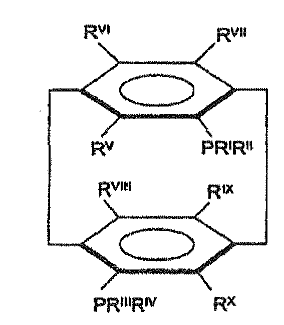 dans laquelle,
R^{I}, R^{II}, R^{III} et R^{IV} représentent indépendamment les uns des autres alkyle, cycloalkyle, hétérocycloalkyle, aryle ou hétaryle, et
R^{V}, R^{VI}, R^{VII}, R^{VIII}, R^{IX} et R^{X} représentent indépendamment les uns des autres hydrogène, alkyle, alkylène-OH, alkylène-NE¹E ², alkylène-SH, alkylène-OSiE³E⁴, cycloalkyle, hétérocycloalkyle, aryle, hétaryle, OH, SH, oxyde de polyalkylène, polyalkylène imine, alcoxy, halogène, COOH, carboxylate, SO₃H, sulfonate, NE¹E², nitro, alcoxycarbonyle, acyle ou cyano, E¹, E², E³ et E⁴ représentant à chaque fois des radicaux identiques ou différents, choisis parmi hydrogène, alkyle, cycloalkyle, aryle et alkylaryle,
étant utilisé en tant que catalyseur pour l'hydrogénation, avec obtention d'un mélange d'énantiomères enrichi en un énantiomère,
- le mélange d'énantiomères obtenu lors de l'hydrogénation est soumis à une cristallisation par ajout d'un agent de formation de sels basique dans un solvant pour l'enrichissement énantiomérique supplémentaire, et le solide ainsi formé, enrichi en un stéréoisomère, est isolé, et
- l'isomère isolé est éventuellement soumis à une protonation ou un échange de cations avec obtention du composé optiquement actif de formule I.

2. Procédé selon la revendication 1, dans lequel un mélange d'isomères cis/trans qui contient au moins 50 % en poids, de préférence au moins 60 % en poids, notamment au moins 70 % en poids de l'isomère cis, est utilisé pour l'hydrogénation.

3. Procédé selon l'une quelconque des revendications précédentes, dans lequel un mélange d'isomères cis/trans qui contient au moins 1 % en poids, de préférence au moins 5 % en poids, notamment au moins 10 % en poids de l'isomère trans, est utilisé pour l'hydrogénation.

4. Procédé selon la revendication 1, dans lequel R^{I}, R^{II}, R^{III} et R^{IV} représentent indépendamment les uns des autres phényle, tolyle, méthoxyphényle, xylyle ou méthoxyxylyle.

5. Procédé selon l'une quelconque des revendications 1 à 4, dans lequel un des radicaux R^{V}, R^{VI} et R^{VII} et/ou un des radicaux R^{VIII}, R^{IX} et R^{X} représentent un radical différent de l'hydrogène et le ou les radicaux différents de l'hydrogène sont choisis parmi C₁-C₆-alkyle, C₁-C₄-alkylène-OH, C₁-C₄-alkylène-OSi (C₁-C₄-alkyle) ₂, C₁-C₄-alcoxy, C₁-C₄-alkylène-OC(alkyle)₃ et C₁-C₄-alkylène-OC (aryle) ₃.

6. Procédé selon l'une quelconque des revendications 1 à 5, dans lequel le catalyseur comporte au moins un ligand choisi parmi les composés de formules

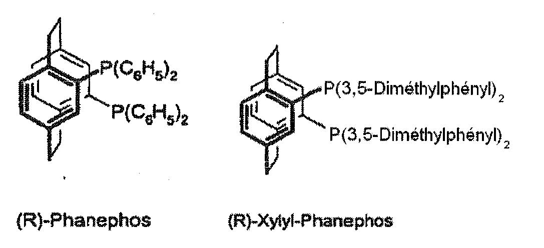

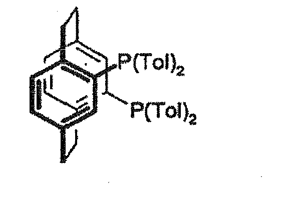

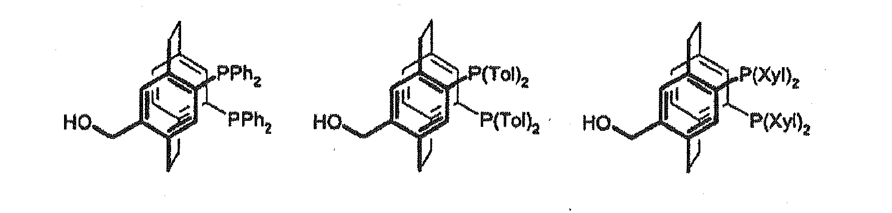

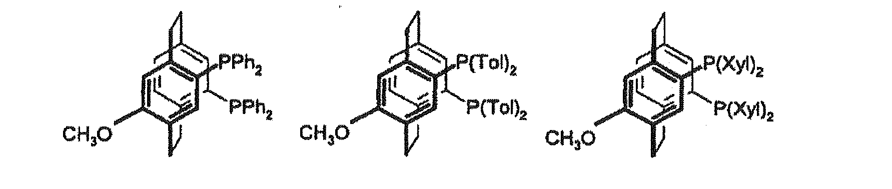

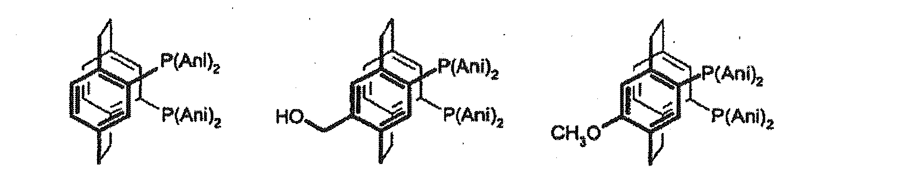

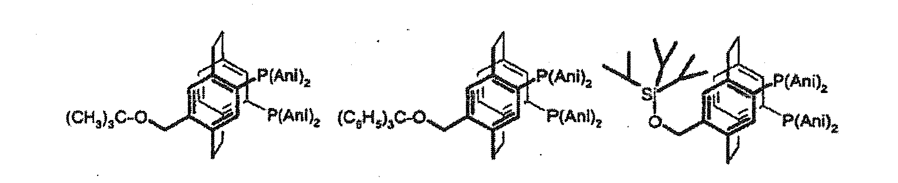

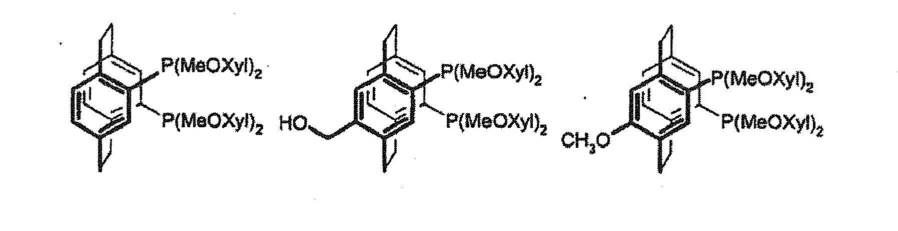

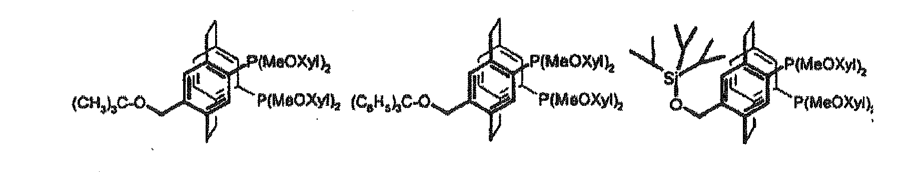

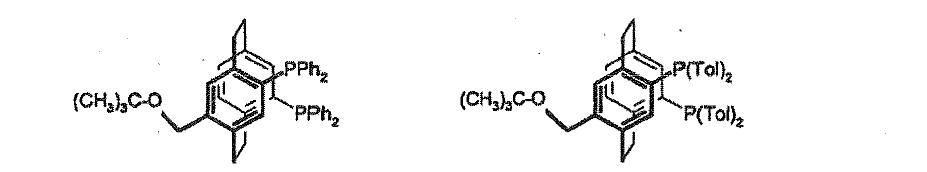

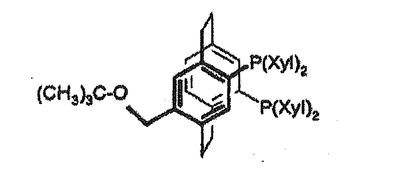

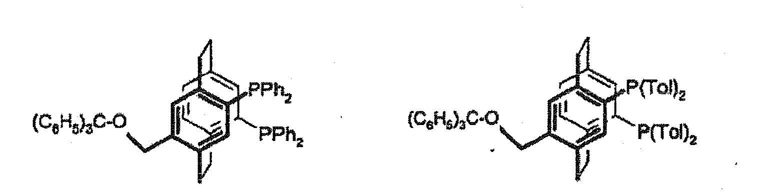

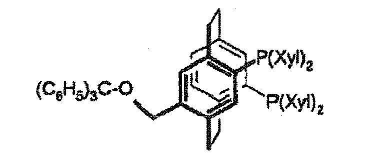

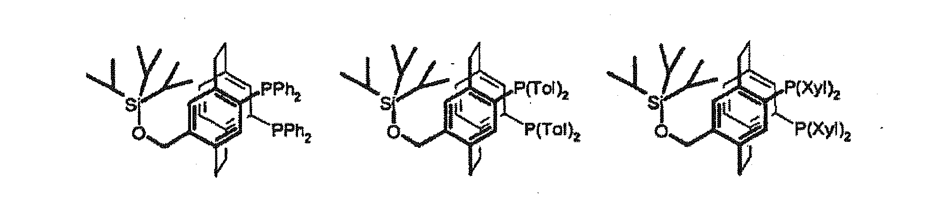

7. Procédé selon l'une quelconque des revendications 1 à 6, dans lequel le catalyseur comporte au moins un ligand choisi parmi les composés de formules

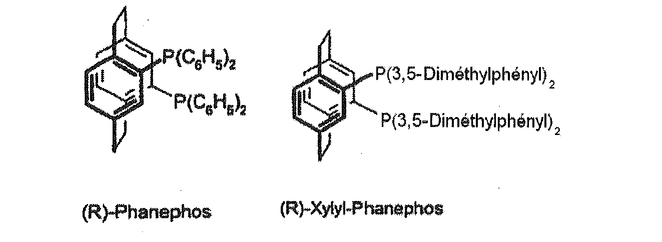

8. Procédé selon l'une quelconque des revendications précédentes, dans lequel l'hydrogénation a lieu en continu.

9. Procédé selon la revendication 8, dans lequel
i) un mélange d'isomères de composés de formule générale II et de l'hydrogène sont introduits dans une première zone de réaction et mis en réaction en présence d'un catalyseur d'hydrogénation chiral jusqu'à une conversion partielle,
ii) un courant est extrait de la première zone de réaction et hydrogéné dans au moins une zone de réaction supplémentaire.

10. Procédé selon l'une quelconque des revendications précédentes, dans lequel l'agent de formation de sels utilisé pour la cristallisation est choisi parmi les composés basiques achiraux.

11. Procédé selon la revendication 10, dans lequel l'agent de formation de sels est choisi parmi l'ammoniac, les amines primaires, les hydroxydes alcalins et les hydroxydes alcalino-terreux.

12. Procédé selon l'une quelconque des revendications 10 ou 11, dans lequel l'ammoniac ou LiOH est utilisé en tant qu'agent de formation de sel et l'isopropanol en tant que solvant pour la cristallisation.

13. Procédé selon l'une quelconque des revendications précédentes, dans lequel le solide isolé après la cristallisation présente une valeur ee d'au moins 98 %.

14. Procédé selon l'une quelconque des revendications précédentes, dans lequel un composé optiquement actif de formule I ayant la configuration absolue suivante est obtenu 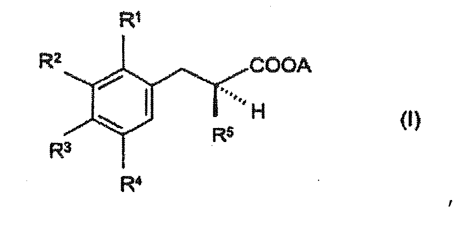 R¹ à R⁵ et A ayant les significations données dans la revendication 1.

15. Procédé de fabrication de composés optiquement actifs de formule générale III 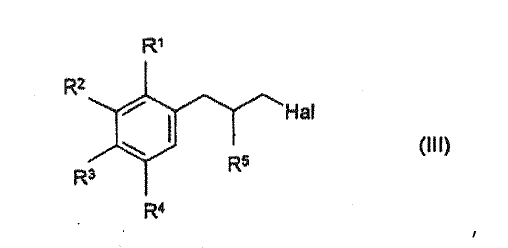 dans laquelle R¹ à R⁵ ont les significations données dans la revendication 1 et Hal représente Cl, Br ou I, selon lequel
a) un aldéhyde aromatique de formule générale V 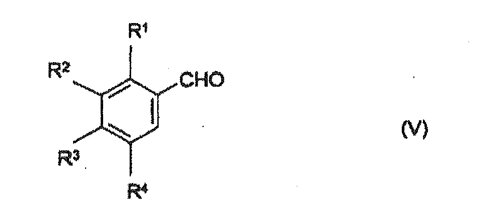 dans laquelle R¹ à R⁴ ont les significations données dans la revendication 1, est mis en réaction avec un ester d'acide carboxylique de formule générale VI
R⁵-CH₂-COOR⁷ (VI)
dans laquelle R⁵ a les significations données dans la revendication 1 et R⁷ représente alkyle, cycloalkyle, aryle ou alkylaryle, avec obtention de composés de formule générale VII 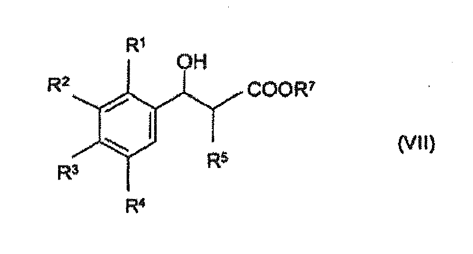
b) dans les composés de formule VII, le groupe hydroxy est transformé en un meilleur groupe partant et soumis à une élimination avec obtention de composés de formule générale VIII 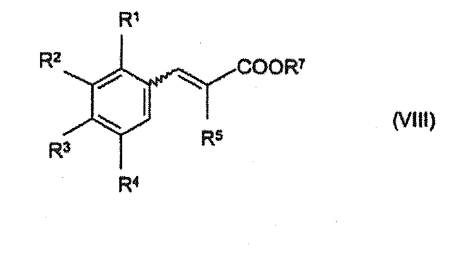
c) les composés de formule VIII sont soumis à une hydrolyse d'ester avec obtention de composés de formule générale II 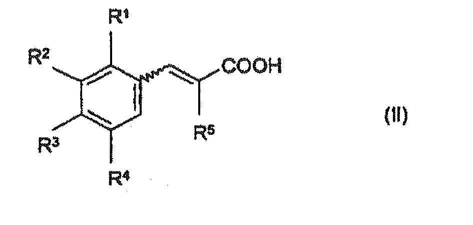
d) les composés de formule II sont soumis à une hydrogénation énantiosélective en présence d'un catalyseur d'hydrogénation chiral tel que défini dans l'une quelconque des revendications 1 ou 4 à 7, avec obtention d'un mélange d'énantiomères enrichi en un énantiomère,
e) le mélange d'énantiomères obtenu lors de l'hydrogénation de l'étape d) est soumis à une cristallisation par ajout d'un agent de formation de sels basique dans un solvant pour l'enrichissement énantiomérique supplémentaire, et le solide ainsi formé, enrichi en un stéréoisomère, est isolé,
f) l'isomère isolé à l'étape e) est éventuellement soumis à une protonation ou un échange de cations avec obtention du composé optiquement actif de formule I,
g) dans le cas où le radical A du composé de formule I représente un équivalent cationique différent de l'hydrogène et de cations métalliques, celui-ci est soumis à une protonation,
h) l'acide ou son sel métallique est soumis à une réduction avec obtention d'un alcool de formule générale IV 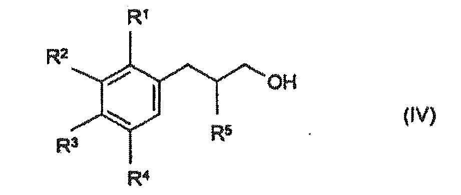 et
i) l'alcool de formule IV est soumis à une halodéshydroxylation avec obtention du composé optiquement actif de formule III

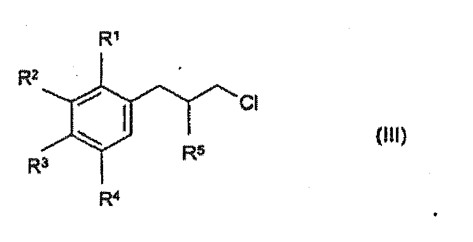

16. Composé optiquement actif de formule générale I 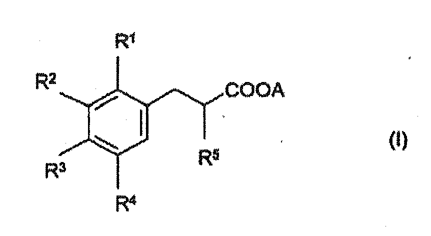 dans laquelle R¹ à R⁵ ont la signification donnée dans la revendication 1 et A représente un cation dérivant de l'ammoniac, d'amines primaires, de métaux alcalins et de métaux alcalino-terreux.

17. Composé selon la revendication 16, dans lequel R⁵ représente un radical C₃-C₈-alkyle ramifié, notamment isopropyle.

18. Composé selon l'une quelconque des revendications 16 ou 17, de formule

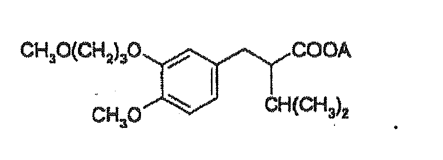

19. Composé selon l'une quelconque des revendications 16 à 18, dans lequel A représente NH₄⁺ ou Li⁺.
